(19) Europäisches Patentamt / European Patent Office / Office européen des brevets

(11) **EP 3 003 360 B1**

(12) **EUROPEAN PATENT SPECIFICATION**

(45) Date of publication and mention
of the grant of the patent:
**07.10.2020 Bulletin 2020/41**

(21) Application number: **14859866.7**

(22) Date of filing: **05.11.2014**

(51) Int Cl.:
***A61K 38/54*** *(2006.01)*   ***C12N 9/94*** *(2006.01)*

(86) International application number:
**PCT/US2014/063984**

(87) International publication number:
**WO 2015/069677 (14.05.2015 Gazette 2015/19)**

(54) **HIGH POTENCY PANCREATIN PHARMACEUTICAL COMPOSITIONS**

HOCHWIRKSAME PHARMAZEUTISCHE PANKREATINZUSAMMENSETZUNGEN

COMPOSITIONS PHARMACEUTIQUES DE PANCRÉATINE DE HAUTE PUISSANCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.11.2013 US 201361900092 P**

(43) Date of publication of application:
**13.04.2016 Bulletin 2016/15**

(60) Divisional application:
**19201877.8 / 3 613 429**

(73) Proprietor: **Allergan Pharmaceuticals
International Limited
Dublin 17, D17 E400 (IE)**

(72) Inventors:
• **PERRETT, Stephen**
  **Princeton, NJ 08540 (US)**
• **BECKER, Robert**
  **D-88400 Biberach an der Riß (DE)**
• **BOLTRI, Luigi**
  **I-20041 Agrate Brianza (MB) (IT)**
• **GHIDORSI, Luigi**
  **I-20129 Milano (IT)**
• **ARZUFFI, Paola**
  **I-24042 Capriate San Gervasio (BG) (IT)**
• **PIRONTI, Vincenza**
  **20873 Milano (IT)**

(74) Representative: **Lee, Nicholas John et al
Kilburn & Strode LLP
Lacon London
84 Theobalds Road
London WC1X 8NL (GB)**

(56) References cited:
EP-A2- 0 115 023      WO-A1-2004/074470
WO-A2-2015/019198     CN-A- 103 060 296
GB-A- 732 951         GB-A- 2 234 973
US-A- 5 075 231       US-A- 5 750 104
US-A1- 2004 057 944   US-A1- 2006 198 838

• JI-HWAN HWANG ET AL: "Selective Precipitation of Proteins from Pancreatin Using Designed Antisolvents", INDUSTRIAL & ENGINEERING CHEMISTRY RESEARCH, vol. 46, no. 12, 1 June 2007 (2007-06-01), pages 4289-4294, XP055169811, ISSN: 0888-5885, DOI: 10.1021/ie061280f

## Description

### Field

The present invention is directed to a process of producing mammalian-derived HA-pancreatin enzymes. Disclosed herein are high potency pharmaceutical compositions including high activity pancreatin (HA-pancreatin) enzymes.. Also disclosed herein are compositions or dosage forms, and methods for their use.

### Background

Exocrine pancreatic insufficiency (EPI), of which the FDA estimates more than 200,000 Americans suffer, involves a physiological disorder wherein individuals are incapable of properly digesting food due to a lack of digestive enzymes made by their pancreas. This loss of digestive enzymes leads to disorders such as the maldigestion and malabsorption of nutrients, which lead to malnutrition and other consequent and undesirable physiological conditions associated therewith. These disorders are common for those suffering from cystic fibrosis (CF) and other conditions which compromise the exocrine function of the pancreas, such as pancreatic cancer, pancreatectomy, and pancreatitis. The malnutrition can be life threatening if left untreated, particularly in the case of infants and CF patients. The disorder can lead to impaired growth, a compromised immune response, and shortened life expectancy.

Digestive enzymes, such as pancrelipase enzymes and other pancreatic enzyme products (PEPs) can be administered to at least partially remedy EPI. The administered digestive enzymes allow patients to more effectively digest their food.

The pancrelipase enzymes used for treating EPI are mainly a combination of three enzyme classes: lipase, amylase, and protease, together other enzymes including elastases, phospholipases, and cholesterases, amongst others, and various co-factors and coenzymes. These enzymes are produced naturally in the pancreas and are important in the digestion of fats, proteins and carbohydrates. The enzymes catalyze the hydrolysis of fats into glycerol and fatty acids, starch into dextrin and sugars, and proteins into amino acids and derived substances. Digestion is, however, a complex process involving many other enzymes and substrates that contribute to correct digestive functioning and in producing the full range of digestive products.

Pancrelipase enzymes may be prepared from porcine pancreatic glands. Other pancrelipase sources include bovine pancreatic glands, and/or pancreatic juices. The natural mammalian source of these enzymes results in a product with an enzyme composition which is similar to that secreted by the human pancreas. Other non-mammalian sources can also be used, for example, those described in U.S. 6,051,220, U.S. 2004/0057944, 2001/0046493, and/or WO2006044529.

While the pancrelipase-containing products can offer an effective therapy, there are issues therewith. A need for multiple (4-9) relatively large capsules (high pill load) with every meal decreases a patient's adherence to dosing. Potential microbial and viral contamination consequent to a high pill load is also noted to be undesirable. All of these issues are linked to the enzyme extract being less pure. The purity issue is a consequence of enzyme extraction procedures having been in place for many years, involving the formation of a coarse aqueous blend or slurry, precipitation with alcohol, centrifugation and filtration. Such extraction processes yield final products that may possess as little as 25% protein. For example, Lipase, an important enzyme in terms of efficacy in these pancrelipase extracts, has an activity in the region of 100 IU/mg. This contrasts with the activity of pure porcine lipase, which has an activity of approximately 25,000 IU/mg (such as pure porcine lipase available from Sigma Aldrich). Using this approximation as a basis for calculation, the products can be estimated to contain less than 0.5% active lipase. Furthermore, the additional consequence of the presence of excess inactive material is that any infectious contamination may be inevitably a part of this bulk and consequently also ingested along with the desirable active components of the mixture. The excess of inactive materials also interfere with techniques aimed at reducing bio-burden, for example through clogging of membrane filters or filtration columns and shielding the extract from ionizing radiation useful for decreasing its potential infectious burden.

A number of pure single enzymes and a mixture of three single enzymes, in one case, have entered clinical development for the treatment of EPI. These are recombinant bile salt stimulated lipase (BSSL) (EXINALDA™/KIOBRINA®); a recombinant human lipase contained in mothers' milk MERISPASE®; a recombinant canine gastric lipase MS1819; a recombinant lipase and liprotamase; a mixture consisting of a chemically cross-linked recombinant bacterial lipase; and a protease and an amylase extracted from microbial sources. All of these experimental therapies have so far failed to demonstrate a level of treatment efficacy that is comparable to that of commercial enzyme extracts from porcine pancreas such as ZENPEP® or ULTRESA®. Likewise, an FDA advisory board meeting on the 12th January 2011 voted against approval of liprotamase owing to its lower efficacy in terms of increase in coefficient of fat absorption (CFA) in comparison to that previously obtained with pancrelipase extract products.

There is a clear need for a product which is more concentrated and purified in comparison to existing pancrelipase-containing products, yet which maintains its efficacy for the treatment of EPI, as this would allow better, more convenient and potentially safer products to be produced. There are a number of literature reports that describe the use of pancrelipase as a starting material for the isolation of proteases, lipases or amylases. However, there are no reports of pancrelipase of the type that is found in PEPs or similar products being purified for the purposes of creating an improved product for therapeutic use. In each case, prior efforts have been to purify a particular enzyme or enzyme fraction over others or to remove certain components without materially increasing overall enzyme activity. In all cases there has also been no direction to produce a HA-pancreatin product for use as a therapeutic agent.

Prior art methods for protein purification either aims to extract and isolate a simple protein-rich fraction, as exemplified by pancrelipase, or to separate single proteins or single classes of proteins, e.g., lipases or proteases.

For example, Hwang et al. (Ind. Eng. Chem. Res. 2007, 46, 4289) discloses a relationship between pancreatic enzymes solubility and solvent polarity and reports about the selective precipitation of lipase, protease and amylase from pancreatic proteins. Hwang et al. shows that pancreatin precipitation is enhanced when a solvent with reduced polarity is used and that it is maximized when the solvent has a Hildebrand solubility parameter below 28 $(MPa)^{0.5}$. Selective precipitation of amylase and protease increases with decreasing solvent polarity below 34 $(MPa)^{0.5}$, whereas selective precipitation of lipase is independent of solution polarity, and not more than 65% of lipase present in the mixture is recovered. From these results there is no incentive to purify a mixture of pancreatic enzymes together to obtain a HA-pancreatin, and there is no incentive for those skilled in the art to preserve a mixture of different enzyme classes during purification. The fact that there has been no attempt to purify the pancrelipase that has been used in therapeutic products in well over 60 years is a strong indicator that the benefits of doing this have not been envisaged or appreciated. All attempts to improve pancrelipase products have focused on single enzymes from non-pancreatic sources, further emphasizing that the use pancrelipase as a source for purer and/or more concentrated enzymes for the manufacture of improved products has not been previously appreciated.

There is no incentive or reason to purify pancrelipase, as it is currently used in pharmaceutical and cleaning applications, with the aim of producing a product with a substantially similar qualitative and quantitative profile of enzyme activity with several fold higher enzyme concentration. Indeed, the current products have fulfilled their roles adequately and as such have remained substantially unchanged for over 60 years, and there appears to be no descriptions in the art of the markedly improved products or associated preparation processes described herein. Those products containing pure enzymes for the treatment of EPI have all been based on single enzymes or, in one case, a blend of three pure and chemically modified single enzymes, from recombinant technology or microbial sources. There has been no effort to purify a mixture including protease, lipase and amylase from the crude pancreas gland extracts that are used for the treatment of EPI, cleaning and tissue digestion. Likewise, there has been no incentive or reports of the enzymes from a pancreatic source being purified individually and then later recombined. Such an approach is counter to the aim of achieving an isolated enzyme or enzyme class.

## Summary

The present invention is directed to a process for the preparation of mammalian-derived HA-pancreatin including lipases, proteases and amylases, having specific lipase activity of at least 120 IU/mg, comprising

a1.1) suspending starting mammalian pancreatin and precipitating a first insoluble portion in a solvent having a Hildebrand solubility parameter of 38 $(MPa)^{0.5}$;
a1.2) separating the soluble portion of step a1.1 from the first insoluble portion;
a1.3) precipitating a second insoluble portion by adding to the soluble portion of step a1.2 at least one organic solvent to obtain a mixture having a Hildebrand solubility parameter of 36;
a2) separating the second insoluble portion of step a 1.3 from the soluble portion;
a3.1) drying the first insoluble portion of step a1.2;
a3.2) drying the second insoluble portion of step a2;
a4) mixing together the first insoluble portion of step a3.1 with the second insoluble portion of step a3.2, wherein

   i) said solvent used in step a1.1 is a mixture of acetone and buffer solution having a pH of 7,
   ii) the process temperature is 4°C,
   iii) the mixture of step a1.3 is a mixture of acetone and buffer solution having a pH of 7, and
   iv) the pancreatin of step a1.1 is in an amount of 0.1 g/ml.

Preferred features of the invention are set out in the dependent claims herein.

The present disclosure is directed to HA-pancreatin enzymes and high potency pharmaceutical compositions or dosage forms thereof. The present disclosure is also directed to high yield process of producing HA-pancreatin and methods for the use of such product.

## Brief Description of the Figures

Figure 1 shows the UV absorption spectrum of pancreatin extract.

Figure 2 shows the UV Absorption Spectrum of the supernatant (A) and the re-dissolved precipitate (B) resulting from the ammonium sulfate precipitation of pancreatin described in Example 15.

Figure 3 shows the UV absorption spectrum of the unwashed and washed ammonium sulfate precipitates of Example 15.

Figure 4 shows the elution profile of the ammonium sulfate fraction of Example 15. Fractions 4-8 show absorption at 280 nm, indicating the presence of protein.

Figure 5 shows the UV absorption spectrum of fractions 6 and 10 of Example 15.

Figure 6 shows the elution profile of the ammonium sulfate fraction of Example 17.

## Detailed Description

The present disclosure is directed to a high activity-pancreatin (HA-pancreatin) that includes essential enzyme classes having effective and significantly high therapeutic activity, more particularly, also having decreased bio-burden of unnecessary biological components and/or undesirable potentially infectious components. The present invention is directed to a process for producing mammalian-derived HA-pancreatin, more particularly, in a very high yield. The HA-pancreatin enables the formulation of smaller and more convenient dosage forms, particularly dosage forms that may be delivered as a single pill, small particle dosage forms for suspension and dosage forms which can be combined with other therapeutic or useful ingredients in a single dosage unit. The compositions of the present disclosure may be of particular value to patients suffering from EPI, such as cystic fibrosis patients. The present disclosure would also be useful for formulating into formulations where the age or condition of the patient may require alternative administrative forms other than a capsule, e.g., suspension, and/or particles. A more concentrated, and hence smaller, dosage form, unit or particle would be of great value for this patient group. The present disclosure also enables the application of technologies designed to reduce or remove unnecessary or undesirable biological constituents such as microbes and any associated toxins for the reasons outlined above.

The present disclosure is directed to HA-pancreatin enzymes (HA-pancreatin) and high potency pharmaceutical compositions thereof. The HA-pancreatin may be porcine derived. The HA-pancreatin includes lipase, proteases, and amylase and has a specific lipase activity of at least about 120, or at least about 150, or at least about 200, or at least about 400, or at least about 500 IU/mg.

The term "digestive enzyme" used herein denotes an enzyme in the alimentary tract which breaks down the components of food so that they can be taken or absorbed by the organism. Non-limiting examples of digestive enzymes include pancrelipase enzymes (also referred to as pancrelipase or pancreatin), lipase, co-lipase, trypsin, chymotrypsin, chymotrypsin B, pancreatopeptidase, carboxypeptidase A, carboxypeptidase B, glycerol ester hydrolase, phospholipase, sterol ester hydrolase, elastase, kininogenase, ribonuclease, deoxyribonuclease, α-amylase, papain, chymopapain, glutenase, bromelain, ficin, β-amylase, cellulase, β-galactosidase, lactase, sucrase, isomaltase, and mixtures thereof.

The term "pancreatic enzyme" as used herein refers to any one of the enzyme types present in the pancreatic secretion, such as amylase, lipase, protease, or mixtures thereof, or any extractive of pancreatic origin having enzymatic activity, such as pancreatin.

The terms "pancrelipase enzymes" or "pancrelipase" or "pancreatin" denotes a mixture of several types of enzymes, including amylase, lipase, and protease enzymes. Pancrelipase enzyme is commercially available, for example, from Nordmark Arzneimittel GmbH, or Scientific Protein Laboratories LLC.

The term "API" is used herein to denote "digestive enzymes" or "pancrelipase enzymes" or "pancreatin".

The term "lipase" denotes an enzyme that catalyzes the hydrolysis of lipids to glycerol and simple fatty acids. Examples of lipases suitable include, but are not limited to, animal lipase (e.g., porcine lipase), bacterial lipase (e.g., Pseudomonas lipase and/or Burkholderia lipase), fungal lipase, plant lipase, recombinant lipase (e.g., produced via recombinant DNA technology by a suitable host cell, selected from any one of bacteria, yeast, fungi, plant, insect or mammalian host cells in culture, or recombinant lipases which include an amino acid sequence that is homologous or substantially identical to a naturally occurring sequence, lipases encoded by a nucleic acid that is homologous or substantially identical to a naturally occurring lipase-encoding nucleic acid, etc.), synthetic lipase, chemically-modified lipase, and mixtures thereof. The term "lipids" broadly includes naturally occurring molecules including fats, waxes, sterols, fat-soluble vitamins (such as vitamins A, D, E and K), monoglycerides, diglycerides, triglycerides, phospholipids, etc.

The term "amylase" refers to glycoside hydrolase enzymes that break down starch, for example, $\alpha$-amylases, $\beta$-amylases, $\gamma$-amylases, acid $\alpha$-glucosidases, salivary amylases such as ptyalin, etc. amylases suitable for use in the present disclosure include, but are not limited to, animal amylases, bacterial amylases, fungal amylases (e.g., Aspergillus amylase, for example, Aspergillus oryzae amylase), plant amylases, recombinant amylases (e.g., produced via recombinant DNA technology by a suitable host cell, selected from any one of bacteria, yeast, fungi, plant, insect or mammalian host cells in culture, or recombinant amylases which include an amino acid sequence that is homologous or substantially identical to a naturally occurring sequence, amylases encoded by a nucleic acid that is homologous or substantially identical to a naturally occurring amylase-encoding nucleic acid, etc.), chemically modified amylases, and mixtures thereof.

The term "protease" refers generally to enzymes (e.g., proteinases, peptidases, or proteolytic enzymes) that break peptide bonds between amino acids of proteins. Proteases are generally identified by their catalytic type, e.g., aspartic acid peptidases, cysteine (thiol) peptidases, metallopeptidases, serine peptidases, threonine peptidases, alkaline or semi- alkaline proteases, neutral and peptidases of unknown catalytic mechanism. Non-limiting examples of proteases suitable for use in the present disclosure include serine proteases, threonine proteases, cysteine proteases, aspartic acid proteases (e.g., plasmepsin) metalloproteases and glutamic acid proteases. In addition, proteases suitable for use in the present disclosure include, but are not limited to, animal proteases, bacterial proteases, fungal proteases (e.g., an Aspergillus melleus protease), plant proteases, recombinant proteases (e.g., produced via recombinant DNA technology by a suitable host cell, selected from any one of bacteria, yeast, fungi, plant, insect or mammalian host cells in culture, or recombinant proteases, which include an amino acid sequence that is homologous or substantially identical to a naturally occurring sequence, proteases encoded by a nucleic acid that is homologous or substantially identical to a naturally occurring protease-encoding nucleic acid, etc.), chemically modified proteases, and mixtures thereof.

The pancrelipase enzymes of the composition of the present disclosure can include one or more lipases (i.e., one lipase, or two or more lipases), one or more amylases (i.e., one amylase, or two or more amylases), one or more proteases (i.e., one protease, or two or more proteases), and mixtures of these enzymes in different combinations and ratios.

Lipase activities in the compositions of present disclosure can be from about 650 to about 100,000 IU (USP method). It can be from about 675 to about 825 IU, from about 2,500 to about 28,000 IU, from about 2,700 to about 3,300 IU, from about 4,500 to about 5,500 IU, from about 8,000 to about 11,000 IU, from about 13,500 to about 16,500 IU, and from about 18,000 to about 22,000 IU, from about 22,500 to about 27,500 IU, from about 36,000 to about 44,000 IU, and all ranges and subranges there between.

The compositions of the present disclosure preferably can contain at least about 650 IU (USP method), at least about 9,000, even more preferably they contain about 20,000, about 40,000, about 60,000, about 80,000, or about 100,000 IU units lipase per dosage unit.

The HA-pancreatin composition according to the present disclosure may be in powder form or may be in compacted form, e.g., a tablet, or may include a plurality of coated and/or uncoated particles. The particles may include a core coated with at least one enteric coating, wherein said coating contains an enteric polymer. The above composition besides the coated particles may also include uncoated particles of pancrelipase. In particular, the particles are minitablets, microtablets, microparticles, microspheres, microcapsules, and/or micropellets. The particles can have diameters up to about 5 mm. They can have any suitable particle size or shape. For example, the particles can have a particle size range from about 25-5,000 $\mu$m. For example, they can be in the form of "minitablets" which have a nominal particle diameter in the range of about 2-5 mm, or they can be "microtablets" which have nominal particle diameters of less than about 2 mm, for example about 1-2 mm. The particles can have an average particle size of less than about 800 $\mu$m, preferably less than about 500 $\mu$m, more preferably less than about 200 $\mu$m. The particles may have a volume diameter (d(v,0.1)) (defined as the diameter where 10% of the volume distribution is below this value and 90% is above this value) of not less than 400 $\mu$m and a volume diameter d(v,0.9) (defined as the diameter where 90% of the volume distribution is below this value and 10% is above this value) of not more than about 800 $\mu$m.

Where pancrelipase cores are surrounded by an enteric coating, the coating acts as a barrier protecting the medication from the acidic environment of the stomach and substantially preventing the release of the medication before it reaches the small intestine. Suitable combinations of enteric coating compositions with other coating compositions can be used to provide the desired type of control over drug release or therapeutic effects. The enteric coating includes at least one enteric polymer and further excipients. The phrase "enteric polymer" means a polymer that protects the digestive enzymes from gastric contents, for example, a polymer that is stable at acidic pH, but can break down rapidly at higher pH, or a polymer whose rate of hydration or erosion is slow enough to ensure that contact of gastric contents with the digestive enzymes is relatively minor while it is in the stomach, as opposed to the remainder of the gastrointestinal tract.

Non-limiting examples of enteric polymers include cellulose acetate phthalate, hydroxypropylmethylcellulose phthalate, hydroxypropylmethylcellulose acetate succinate, polyvinylacetate phthalate, copolymers of methacrylic acid, esters of methylmethacrylate, methylmethacrylate copolymers, and methacrylic acid/ methylmethacrylate copolymers, methacrylic acid- ethyl acrylate copolymer (1:1), shellac, and ethyl cellulose. These polymers are commercially available with different brand names, such as: Cellacefate (cellulose acetate phthalate), EUDRAGIT® L100, S100, L30D, FS30D, L100-55, L30D55 (copolymers of methacrylic acid), AQUATERIC® (cellulose acetate phthalate), AQOAT® (hydroxypropylmethylcellulose acetate succinate), and HP55® (hydroxypropylmethylcellulose phthalate). The enteric coating may further include other excipients such as talc. Preferably the enteric coating includes: 10-20 wt. % of at least one enteric polymer, wherein each said wt. % is based on the total weight of the coated particles. The coating may further include a lipophilic agent, such as a C6 - C30 lipophilic low molecular weight molecule selected from the aliphatic carboxylic acids and alcohols, preferably a C14 - C18 carboxylic acid or alcohol, such as stearic acid, myristic acid, myristic alcohol, or stearyl alcohol. Other optional ingredients of the coating are plasticizer, anti-tacking agents (such as talc, magnesium stearate, colloidal silicon dioxide and combinations thereof; further optionally a low viscosity ethylcellulose). Non-limiting examples of suitable plasticizers include triacetin, tributyl citrate, tri-ethyl citrate, acetyl tri-n-butyl citrate, diethyl phthalate, dibutyl sebacate, polyethylene glycol, polypropylene glycol, castor oil, acetylated mono- and di-glycerides, cetyl alcohol, and mixtures thereof. The preferred plasticizer is a non-phthalate plasticizer or mixtures thereof.

The HA-pancreatin coated or uncoated particles may be prepared according to known processes. For example, the micropellet cores may be prepared by adding a suitable binder to HA-pancreatin followed by extrusion in the presence of a suitable solvent and subsequent spheronization. Controlled spheronization may be applied to generate HA-pancreatin particles with small size. Spray coating, powder layering and fluid bed technologies may be used for preparing beads through coating an inert core. Coacervation processes may also be useful for the preparation of coated pancrelipase particles.

Direct compression may be used to prepare excipient-free compacted tablets. The tablet may display gastro-resistance owing to the in situ formation of a hydrophobic coating layer on contact with gastric fluids. The compositions including the HA-pancreatin may be in any form suited to the dosing of a therapeutic agent containing digestive enzymes, such as for example, they may be in the form of powders, pellets, microspheres, capsules, sachets, tablets, liquid suspensions and liquid solutions.

In the present disclosure, dosage forms that include HA-pancreatin, in particular, smaller and/or single dosage forms including HA-pancreatin can be prepared. The availability of HA-pancreatin allows a reduction in the size of the capsule and/or enables one to deliver the dose as reduced number of capsules per meal, for example, compared to the typical formulation composition of a 20,000 unit strength ZENPEP® size 0 capsule, which is filled with 250-275 mg of API. An adult patient may take from 4 to 10 such capsules per meal. For an overall daily dosage of 200,000 IU of lipase, a patient now takes 10 capsules and the drug product intake is about 2,500-2,750 mg. A purification of at least about 2 fold constitutes a meaningful improvement, as it would substantially reduce the amount of drug product required, and higher degrees of purification would provide even more benefit. In fact, the HA-pancreatin pharmaceutical dosage form of the present disclosure, which takes the form of an orally administered capsule, which may have a content of about 100-110 mg of drug (vs 250-275 mg) and therefore for an overall daily dosage of 200,000 IU of lipase the patient's drug product intake is about 1,000-1,100 mg (vs 2,500-2,750 mg). Furthermore, with the HA-pancreatin capsule, a size 2 (vs size 0) may be used, thus drastically reducing also the total number of capsules to be administered, or alternatively maintaining a size 0 capsule and properly modulating its content, thus significantly reducing the daily intake. As the EPI treatment is a chronic treatment which frequently begins in infancy, the ability to formulate the pancrelipase such that it can be contained in smaller dosage units and/or taken as a reduced number of dosage units per meal constitutes a significant benefit for patients.

The dosage forms disclosed herein may also include small particle dosage forms. A pancrelipase product with an increased potency per unit volume would overcome significant issues regarding the reduction of the dimension of the beads. The majority of commercial pancrelipase dosage forms are capsules that are filled with pancrelipase beads, which are coated with an enteric polymer. The coating is applied because pancreatic lipase is irreversibly inactivated in acid media. The capsules may be opened and the beads sprinkled on to certain foods, which is an important option for younger patients or those that have difficulty swallowing or coping with the high pill burden. This option does not address the needs of all patients however, as the beads have an appreciable diameter, which may be up to 2 mm. This means that the beads cannot be easily suspended in liquids for babies or patients requiring tube feeding. Attempts to reduce the dimensions of the beads result in large increases in total surface area and consequently much more enteric polymer is needed to effectively cover the increased surface area of the particles. This greatly increases the bulk of the dosage form and the amount of polymer ingested to the point where the bulk of the dosage form further increases pill burden and the levels of coating excipients may exceed established limits placed on their daily intake.

The availability of a HA-pancreatin, with a much reduced bulk, not only allows the entire dose to be contained in a single dosage unit or in a reduced number of dosage units, but also enables the combination of pancrelipase with other compounds. For example, an antacid buffering agent, such as sodium bicarbonate and HA-pancreatin may be combined in a single dosage unit, whereas it would not be possible to contemplate such a combination of pancreatic enzymes and an agent that increases the stomach pH using the current pancrelipase as this would dramatically increase the already very high pill burden as additional capsules/tablets would be required and/or the capsule/tablets would be of excessive size.

The HA-pancreatin of the present disclosure also provides the option of providing a dispersible dosage form without an enteric coating as the buffer would prevent acid-inactivation of the lipase component of the pancreatin. In addition, bicarbonate supplementation may also provide therapeutic benefit as bicarbonate secretion is generally reduced in patients with EPI. This novel dosage form can be formulated for immediate or delayed release and can be dispersed in a liquid medium. This latter property provides a significant advantage for patients requiring a liquid feed as the components would be readily dispersible in the feed or another convenient medium. These combinations can be delivered in a variety of conventional presentations, such as capsules, tablets, sachets, beads and liquids. As mentioned above, the need to coat the pancrelipase dosage forms with an enteric polymer is a consequence of the instability of the lipase enzyme in acidic media. However, if stomach pH is raised, through the use of proton pump inhibitors, then it has been demonstrated that lipase remains active, presumably because it is not exposed to levels of pH that are low enough to inactivate lipase. This approach is not convenient, nor is it necessarily medically desirable, as it increases pill burden and uses an additional chronic medication to overcome the disadvantages of another. Stomach pH can be temporarily neutralized with simple antacids such as sodium bicarbonate and have been shown to be effective in protecting acid labile drugs, such as the PPI omeprazole, which is a component of the drug ZEGERID®. The level of sodium bicarbonate in this drug is 1.1g and the level of omeprazole is either 20 mg or 40 mg and these components are contained in a hard shell capsule.

A single dosage form containing a combination of HA-pancreatin and at least one other active compound, such as $H_2$ antagonists, proton pump inhibitors or bile salts, are also disclosed in the present disclosure.

A product improvement is obtained with the present disclosure. In fact, the preparation of HA-pancreatin results in a reduction in bioburden simply as a result of the reduction in the amount of material carrying this bioburden. In addition, however, the methodologies used for the preparation process are also able to reduce bioburden, and a significantly less encumbered product will be produced as a result. Furthermore, the removal of large quantities of inactive material from the product enables the use of the sterilization techniques that are used for injectable biologics to be applied, e.g., filtration, ultraviolet (UV) light exposure. Again, this represents a significant and unanticipated improvement in product characteristics.

The HA-pancreatin present in the compositions or oral dosage forms disclosed herein is prepared according to the process herein disclosed.

The starting material is mammalian pancreatin. In the present disclosure we may refer to it also by using the terms "API", or "starting pancreatin", or "starting pancreatic enzymes", or "native pancreatin", "starting pancrelipase", or "native pancrelipase".

A convenient starting material is porcine derived pancrelipase; example of starting materials is commercially available material for example from Nordmark Arzneimittel GmbH, or Scientific Protein Laboratories LLC. Similar extracts from bovine or other mammalian sources may also be used. The preferred starting material is porcine derived pancrelipase. The extraction procedures used to produce the crude extract can be summarized as including the following steps: pig glands ground wet; addition of a pancrelipase 'activator'; treatment of the "crude enzyme slurry" with cold and hot isopropanol to precipitate proteins and remove lipids; centrifugation and filtration steps to remove fibrous and to compact and concentrate; vacuum drying of "wet cake"; de-lumped and milling of the "wet cake" for bulk density and particle size. This dry product is the pancreatin used in current products.

The HA-pancreatin of the present disclosure is prepared by treating the starting pancreatin (native pancreatin). It preserves those elements that are key to the efficacy of pancreatic enzyme based products and removes those elements which are non-essential. The material resultant from the process of the present invention is the mammalian-derived HA-pancreatin. This HA-pancreatin may have a specific lipase activity of at least about 120, or at least about 150, or at least about 200, or at least about 400, or at least about 500 IU/mg.

HA-pancreatin of the present disclosure may be obtained by process including the precipitation that may be induced by solvent or by ammonium sulfate.

The HA-pancreatin having a specific lipase activity of at least about 120 IU/mg may be obtained using a process comprising treating pancreatin with a solvent, wherein said solvent has a Hildebrand solubility parameter (SP) comprised between 28 and 45 $(MPa)^{0.5}$, and said solvent is one organic solvent or a mixture of organic solvents, or a mixture of at least one organic solvent and aqueous solvent and the process is carried out at low temperature, preferably at a temperature below room temperature. The precipitation-induced-by-solvent process of the present disclosure is characterized by comprising the suspending of pancreatin, the precipitating of an insoluble portion, the drying of the insoluble portion to obtain the HA-pancreatin.

The HA-pancreatin having a specific lipase activity of at least about 120 IU/mg may be prepared using a process including treating pancreatin with a solvent, wherein said solvent has a Hildebrand solubility parameter (SP) comprised between 28 and 38 (MPa)$^{0.5}$, and said solvent is one organic solvent or a mixture of organic solvents or a mixture of at least one organic solvent and aqueous solvent and the process is carried out at a low temperature, preferably at a temperature below room temperature.

The HA-pancreatin having a specific lipase activity of at least about 120 IU/mg may be prepared using a process including treating pancreatin with a solvent, wherein said solvent has a Hildebrand solubility parameter (SP) comprised between 28 and 34 (MPa)$^{0.5}$, and said solvent is one organic solvent or a mixture of organic solvents or a mixture of at least one organic solvent and aqueous solvent and the process is carried out at a low temperature, preferably at a temperature below room temperature.

The HA-pancreatin having a specific lipase activity of at least about 120 IU/mg may be prepared using a process including treating pancreatin with a solvent, wherein said solvent has a Hildebrand solubility parameter (SP) comprised between 34 and 38 (MPa)$^{0.5}$, and said solvent is one organic solvent or a mixture of organic solvents or a mixture of at least one organic solvent and aqueous solvent and the process is carried out at a low temperature, preferably at a temperature below room temperature.

The HA-pancreatin having a specific lipase activity of at least about 120 IU/mg may be prepared using a process including treating pancreatin with a solvent, wherein said solvent has a Hildebrand solubility parameter (SP) including between 34 and 45 (MPa)$^{0.5}$, and said solvent is one organic solvent or a mixture of organic solvents or a mixture of at least one organic solvent and aqueous solvent and the process is carried out at a low temperature, preferably at a temperature below room temperature. The HA-pancreatin having a specific lipase activity of at least about 120 IU/mg may be prepared using a process including treating pancreatin with a solvent, wherein said solvent has a Hildebrand solubility parameter (SP) including between 38 and 45 (MPa)$^{0.5}$, and said solvent is one organic solvent or a mixture of organic solvents or a mixture of at least one organic solvent and aqueous solvent and the process is carried out at a low temperature, preferably at a temperature below room temperature.

The HA-pancreatin obtained with the process of the present disclosure have a specific lipase activity of at least about 120, or at least about 150, or at least about 200, or at least about 400, or at least about 500 IU/mg. The mammalian-derived HA-pancreatin of the present invention has a specific lipase activity of at least 120 IU/mg.

The Hildebrand solubility parameter is a numerical value that indicates the relative solvency behavior of a specific solvent. It is derived from the cohesive energy density of the solvent, which in turn is derived from the heat of vaporization. Hildebrand values are available from literature sources, such as from Barton Handbook of Solubility Parameters, CRC Press, 1983. The process solvent of the present disclosure is one organic solvent or a mixture of more organic solvents or a mixture of at least one organic solvent and aqueous solvent; the mixture of organic solvent and aqueous solvent may include one or more organic solvent and one or more aqueous solvent. The term "solvent" used in the present specification identifies all possible mixtures described here above, unless otherwise specified. The solvent may have the following solubility values: 45, 42, 40, 38, 36, 35, 34, and 28, the preferred solubility values are 38 and 36.

The organic solvent may be chosen from the group of solvent including n-pentane, n-hexane, n-heptane, diethylether, cyclohexane, carbon tetrachloride, ethylacetate, tetrahydrofuran, chloroform, trichloroethylene, acetone, dimethylformamide, n-propanol, isopropanol, ethanol, dimethylsulfoxide butylalcohol, methanol, acetonitrile, dioxane, and methylenchloride. Preferred organic solvents are acetone, isopropanol, ethanol, and combinations thereof.

The aqueous solvent may be chosen from the group consisting of: water, or buffer solutions. Preferred buffers have pH=7 or pH=4. They may be respectively pH=7: 10 mM phosphate buffer and pH=4.0: 10 mM acetate buffer.

In the present disclosure, the solvent may have a mixture including one or more organic solvent and one aqueous solvent, said mixture has a Hildebrand solubility parameter ranging from 28 to 45 (MPa)$^{0.5}$.

In the present disclosure, the solvent may have a mixture including one or more organic solvent and one aqueous solvent, said mixture has a Hildebrand solubility parameter ranging from 28 to 38 (MPa)$^{0.5}$.

The solvent may have a mixture including one or more organic solvent and one aqueous solvent, said mixture has a Hildebrand solubility parameter ranging from 28 to 34 (MPa)$^{0.5}$

The solvent may have a mixture including one or more organic solvent and one aqueous solvent, said mixture has a Hildebrand solubility parameter ranging from 34 to 38 (MPa)$^{0.5}$

The solvent may have a mixture including one or more organic solvent and one aqueous solvent, said mixture has a Hildebrand solubility parameter ranging from 34 to 45 (MPa)$^{0.5}$

The solvent may have a mixture including one or more organic solvent and one aqueous solvent, said mixture has a Hildebrand solubility parameter ranging from 38 to 45 (MPa)$^{0.5}$

Solubility parameter (SP) of solvent mixture is calculated using the Hildebrand solubility parameters.

The solvent of the present disclosure may have an SP of 38 (MPa)$^{0.5}$ and be a mixture of organic solvent with aqueous solvent. Few examples of such binary solvent having SP= 38 disclosed herein are:

acetone- buffer: volumetric ratio of acetone to pH=7 buffer is 35:65; volumetric ratio of acetone to pH=4.0 buffer is 35:65; where: SP(acetone)=20.2, SP(buffer) =47.9;
ethanol- buffer: volumetric ratio ethanol to pH=7 buffer is 45:55; volumetric ratio of ethanol to pH=4.0 buffer is 45:55; where SP(ethanol)= 26.0, SP(buffer) =47.9;

The solvent used in step a1.1 of the present invention has an SP of 38 $(MPa)^{0.5}$ and is a mixture of acetone and buffer solution having a pH of 7.

A binary solvent with SP =34 $(MPa)^{0.5}$ disclosed herein is: acetone-buffer: volumetric ratio of acetone to pH=7 buffer is 50:50; where SP(acetone)=20.2, SP(buffer) =47.9.

A binary solvent with SP =35 $(MPa)^{0.5}$ disclosed herein is acetone-buffer: volumetric ratio of acetone to pH=7 buffer is 45: 55; where SP(acetone)=20.2, SP(buffer) =47.9.

In the present disclosure (single-step process), the treating of pancreatin with a solvent having SP of 28-45 $(MPa)^{0.5}$ includes the following steps: a1) suspending pancreatin under stirring and precipitating and insoluble portion in a solvent having Hildebrand solubility parameter comprised between 28 and 45 $(MPa)^{0.5}$; a2) separating the insoluble portion (pellet) from the soluble portion (supernatant) of the mixture of step a1; a3) drying the insoluble portion obtained in step a2; and wherein the steps a1-a3 are carried out at temperature below room temperature. Suitable temperature for carrying out the process is 4°C.

In the present disclosure (single-step process), the treating of pancreatin with a solvent having SP of 34-45 $(MPa)^{0.5}$ includes the following steps: a1) suspending pancreatin under stirring and precipitating an insoluble portion in the solvent having Hildebrand solubility parameter comprised between 34 and 45 $(MPa)^{0.5}$; a2) separating at the insoluble portion (pellet) of the mixture of step a1 from the soluble portion (supernatant); a3) drying the insoluble portion of step a2; and wherein the steps a1-a3 are carried out at temperature below room temperature. Suitable temperature for carrying out the process is 4°C.

In the present disclosure (single-step process), the treating of pancreatin with a solvent having SP of 34-38 $(MPa)^{0.5}$ includes the following steps: a1) suspending pancreatin in the solvent under stirring and precipitating an insoluble portion in a solvent having Hildebrand solubility parameter comprised between 34 and 38 $(MPa)^{0.5}$; a2) separating the insoluble portion (pellet) from the soluble portion (supernatant) of the mixture of step a1; a3) drying the insoluble portion of step a2; and wherein the steps a1-a3 are carried out at temperature below room temperature. Suitable temperature for carrying out the process is 4°C.

Step 1a is preferably carried out for about 60 minutes, and the preferred temperature is 4°C. The separating step (step a2) may be carried out by different methods such as centrifugation, sedimentation or filtration. The drying step (a3) can be carried out for example in a high efficiency dryer, vacuum pump, or freeze dryer. Other methods can also be used. The concentration of pancreatin in solvent in step a1 is preferably in an amount comprised between 0.050 and 0.3 g/mL, preferably between 0.065 and 0.1 g/mL, preferably 0.065 or 0.1 g/mL.

Disclosed herein is a single-step process, wherein the solvent has SP of 38 $(MPa)^{0.5}$ and it is a mixture of acetone and pH 7 buffer (such as 10 mM phosphate), and the pancreatin in step a1 is at a concentration of 0.1g/mL.

The solvent (or solvent mixture) having Hildebrand solubility parameter (SP) comprised between 28 and 45 $(MPa)^{0.5}$ is used in the present disclosure for suspending pancreatin and precipitating an insoluble portion. This solvent may be used as single addition for concurrent suspending and precipitating or as two subsequent additions: the first addition for suspending (suspending solvent) and the second addition for precipitating an insoluble portion (precipitating solvent). In this second case, the first addition is preferably an aqueous solvent and the second addition is an organic solvent (or mixture). The solvent mixture that is composed by the aqueous solvent of the first addition (suspending solvent) and the organic solvent of the second addition (precipitating solvent) has solubility parameter comprised between 28 and 45.

In the present disclosure (two-step process), where the solvent is a mixture of organic solvent and aqueous solvent, the pancreatin is first dispersed in the aqueous solvent (suspending solvent) and then the organic solvent (precipitating solvent) is added thereafter. Step a1 may include the following steps: a1.1 suspending pancreatin in the aqueous solvent under stirring (suspending); a1.2 precipitating an insoluble portion by adding to the suspension of step a1.1 the organic solvent or mixture thereof (precipitating). Therefore, this two-step process includes the following steps: a1.1) suspending pancreatin in aqueous solvent under stirring; a1.2) precipitating an insoluble portion by adding to the suspension of step a1.1 the at least one organic solvent or mixture thereof (precipitating); a2) separating the insoluble portion of step a1.2 from the soluble portion; a3) drying the insoluble portion of step a2.

Steps a1-a3 are carried out at a temperature below room temperature. The mixture of step a1.1 is kept under static condition. Duration of time of step a1.1 is from about 10 to about 30 minutes depending upon the scale and the equipment and duration of time of step a1.2 is about 30 minutes.

The pancreatin in aqueous solvent is preferably in amount comprised between 0.050 and 0.3 g/mL, preferably between 0.1 and 0.3 g/mL, preferably 0.1 or 0.3 g/mL. The organic solvent is preferably either ethanol or acetone and the aqueous solvent is preferably a pH=4.0 buffer (such as 10mM acetate buffer) or a pH 7 buffer (such as 10 mM phosphate).

Disclosed herein is a two-step process, wherein the solvent composed by the suspending solvent and the precipitating solvent (solvents used in steps a1.1 and a1.2 respectively) has SP of 38 $(MPa)^{0.5}$ and it is a mixture of acetone (precipitating solvent) and pH 7 buffer (such as 10 mM phosphate) (suspending solvent), and the pancreatin in step a1 is in concentration of 0.1 g/mL.

Disclosed herein is another two-step process, wherein the solvent composed by the suspending solvent and the precipitating solvent (solvents used in steps a1.1 and a1.2 respectively) has SP of 38 $(MPa)^{0.5}$ and it is a mixture of acetone (precipitating solvent) and pH 4 buffer (such as 10 mM acetate buffer) (suspending solvent), and the pancreatin in step a1 is in concentration of 0.1 g/mL.

In the present invention (multi-step process), when the solvent is a mixture of organic solvent and aqueous solvent, the pancrelipase is first dispersed in the aqueous solvent and then the organic solvent is added to the soluble portion of this aqueous dispersion. The process of the present invention comprises step a1) which includes the three steps: a1.1) suspending, a1.2) separating the soluble portion (supernatant) of step a1.1 from the insoluble portion (pellet), a1.3) precipitating. This multi-step process may include the following steps: a1.1) suspending pancreatin in aqueous solvent under stirring; a1.2) separating the soluble portion of step a1.1 from the insoluble portion; a1.3) precipitating an insoluble portion by adding to the soluble portion of step a1.2 the at least one organic solvent (precipitating); a2) separating the insoluble portion of step a1.3 from the soluble portion; a3) drying the insoluble portion of step a2.

Step a1.1 is preferably carried out for about 30 minutes. The mixture of step a1.3 may be kept under static condition for about 15 minutes, and the temperature for this step is 4°C.

The pancreatin in aqueous solvent is preferably in an amount comprised between 0.05 and 0.3 g/mL, preferably from 0.1 to 0.3 g/mL, preferably 0.1 or 0.3 g/mL. The pancreatin of step a1.1) of the present invention is an amount of 0.1 g/ml. Disclosed herein, the SP of the solvent composed of the suspending solvent and the precipitating solvent (solvents used in step a1.1 and step a1.3 respectively) used is preferably 38. The SP of the solvent of step a1.1 of the present invention is 38 and the SP of the solvent of step a1.3 is 36. Disclosed herein the organic solvent is preferably either ethanol or acetone (precipitating solvent) and the aqueous solvent is preferably a pH=4.0 buffer (such as 10mM acetate buffer) or a pH 7 buffer (such as 10 mM phosphate) (suspending solvent). The solvent of step a1.1 and the mixture of step a1.3 of the present invention is a mixture of acetone and buffer solution having a pH of 7.

In one the multi-step process disclosed herein, the solvent composed by the suspending solvent and the precipitating solvent (solvents used in step a1.1 and step a1.3 respectively) has SP of 38 $(MPa)^{0.5}$ and it is a mixture of acetone (precipitating solvent) and pH 4.0 buffer (such as 10mM acetate buffer) (suspending solvent), and the pancreatin in step a1 is at a concentration of 0.3 g/mL.

In yet another multi-step process disclosed herein, the solvent composed of the suspending solvent and the precipitating solvent (solvents used in steps a1.1 and a1.3 respectively) has SP of 38 $(MPa)^{0.5}$ and it is a mixture of ethanol (precipitating solvent) and pH 4.0 buffer (such as 10mM acetate buffer) (suspending solvent), and the pancreatin in step a1 is at a concentration of 0.3 g/mL.

In yet another multi-step process disclosed herein, the solvent composed of the suspending solvent and the precipitating solvent (solvents used in steps a1.1 and a1.3 respectively) has SP of 38 $(MPa)^{0.5}$ and it is a mixture of acetone(precipitating solvent) and pH 4.0 buffer (such as 10mM acetate buffer) (suspending solvent), and the pancreatin in step a1 is at a concentration of 0.1 g/mL.

In yet another multi-step process disclosed herein, the solvent composed of the suspending solvent and the precipitating solvent (solvents used in step a1.1 and step a1.3 respectively) has SP of 38 $(MPa)^{0.5}$ and it is a mixture of acetone (precipitating solvent) and pH 7 buffer (such as 10 mM phosphate) (suspending solvent), and the pancreatin in step 1a is at a concentration of 0.3 g/mL.

The separating step (step of single step process and of multi-step process) may be carried out by different methods such as centrifugation or filtration. All process steps of the HA-pancreatin preparation process are carried out under temperature control, which is always below room temperature, at 4°C. Humidity may be also controlled.

In the present disclosure (double precipitation process), the step of treating pancreatin with a solvent having Hildebrand solubility parameter (SP) comprised between 28 and 45 $(MPa)^{0.5}$ may include the following steps: a1.1) suspending pancreatin and precipitating an insoluble portion in a solvent having Hildebrand solubility parameter comprised between 28 and 45 $(MPa)^{0.5}$ (suspending and precipitating); a1.2) separating the soluble portion of step a1.1 from the insoluble portion a1.3) precipitating an insoluble portion by adding to the soluble portion of step a1.2 the at least one organic solvent or mixture thereof, obtaining mixture with lower SP value than the SP value of solvent used of a1.1 (precipitating); a2) separating the insoluble portion of step a1.3 from the soluble portion; a3.1) drying the insoluble portion of step a1.2; a3.2) drying the insoluble portion of step a2; a4) mixing together the insoluble portion of step a3.1 with the insoluble portion of step a3.2.

The SP value of solvent of step a1.3 is different from the value of the SP of solvent used in a1.1; it is always at least about two units lower that the SP value of the solvent in a1.1. For example, when the SP of solvent in a1.1 is 38, then SP of solvent in step 1.3 is 36 or lower, it may be preferably between 27 and 36.

In the present disclosure (double precipitation process), the step of treating pancreatin with a solvent having SP of 38-45 (MPa)$^{0.5}$ may include the following steps: a1.1) suspending pancreatin and precipitating an insoluble portion in a solvent having Hildebrand solubility parameter comprised between 38 and 45 (MPa)$^{0.5}$ (suspending and precipitating); a1.2) separating the soluble portion of step a1.1 from the insoluble portion; a1.3) precipitating an insoluble portion by adding to the soluble portion of step a1.2 the at least one organic solvent or mixture thereof, obtaining Hildebrand solubility parameter comprised between 28 and 36 (precipitating); a2) separating the insoluble portion of step a1.3 from the soluble portion; a3.1) drying the insoluble portion of step a1.2; a3.2) drying the insoluble portion of step a2; a4) mixing together the insoluble portion of step a3.1 with the insoluble portion of step a3.2.

In the present disclosure (double precipitation process), the step of treating of pancreatin with a solvent having SP of 38 (MPa)$^{0.5}$ may include the following steps: a1.1) suspending pancreatin and precipitating an insoluble portion in a solvent having Hildebrand solubility parameter of 38 (MPa)$^{0.5}$ (suspending and precipitating); a1.2) separating the soluble portion of step a1.1 from the insoluble portion; a1.3) precipitating an insoluble portion by adding to the soluble portion of step a1.2 the at least one organic solvent or mixture thereof obtaining Hildebrand solubility parameter of 36 (precipitating); a2) separating the insoluble portion of step a1.3 from the soluble portion; a3.1) drying the insoluble portion of step a1.2; a3.2) drying the insoluble portion of step a2; a4) mixing together the insoluble portion of step a3.1 with the insoluble portion of step a3.2.

Drying steps a3.1 and a.3.2 are carried out under vacuum for 48 hours at room temperature; and wherein the steps a1.1, a1.2, a1.3, a2, are carried out at a temperature below room temperature. Suitable temperature for carrying out the process is 4°C.

The separating step may be carried out by different methods such as centrifugation, sedimentation or filtration. The drying step can be carried out for example in a high efficiency dryer, vacuum pump, or freeze dryer. Other methods can also be used. The concentration of pancreatin in solvent in step a1 is preferably in an amount comprised between 0.05-02 g/mL, preferably 0.1g/mL.

In the double precipitation process of the present invention, the solvent composed by the suspending solvent and the precipitating solvent (solvent used in step a1.1) has SP of 38 (MPa)$^{0.5}$ and it is a mixture of acetone (precipitating solvent) and pH 7 buffer (such as 10 mM phosphate) (suspending solvent), the solvent of step a1.3 has SP of 36 (MPa)$^{0.5}$ is a mixture of acetone (precipitating solvent) and pH 7 buffer (such as 10mM phosphate) (suspending solvent) and the pancreatin in step a1 is in concentration of 50 g/500 mL.

In the double precipitation process of the present invention, the solvent composed by the suspending solvent and the precipitating solvent (solvent used in steps a1.1) has SP of 38 (MPa)$^{0.5}$ and it is a mixture of acetone (precipitating solvent) and pH 7 buffer (such as 10 mM phosphate) (suspending solvent), the solvent of step a1.3 has SP of 36 (MPa)$^{0.5}$ is a mixture of acetone (precipitating solvent) and pH 7 buffer (such as 10mM phosphate) (suspending solvent) and the pancreatin in step a1 is at a concentration of 0.1 g/mL.

Pancreatin extract may be purified by precipitation-induced-by-ammonium sulfate process. Pancreatin may be dispersed in water or a buffer, such as, but not limited to, phosphate buffered saline. Any solids that may be present are removed, either though centrifugation and recovery of the supernatant or through any other suitable method. A suitable amount of a solution of saturated ammonium sulfate is then added to the pancreatin solution so prepared such that the dissolved contents precipitate. The precipitate is recovered through any suitable method, such as, but not limited to, centrifugation and/or filtration. The recovered precipitate is then resolublized in water or a suitable buffer, such as, but not limited to phosphate buffered saline.

Pancreatin may be dispersed in suitable aqueous media, such as PBS and the dispersion incubated on ice for about five to about fifteen minutes with occasional stirring. The dispersion is then centrifuged at 16,000xg for about three to about eight minutes and the supernatant is decanted. A solution of saturated ammonium sulfate is then added to the supernatant for a final concentration of about 50% to about 75% saturated ammonium sulfate. The suspension is then centrifuged at 16,000xg for about three to about eight minutes and the supernatant removed and the pellet resolublized in PBS. The pellet may be washed with saturated ammonium sulfate prior to resolubilization.

The process of the present disclosure may also include sterilization and viral inactivation or viral load reduction of the HA-pancreatin, which may be carried out, for example, by filtration, heating, irradiation (ultraviolet radiation, X-radiation, beta-radiation and gamma-radiation), high pressure treatment and/or alkylation of nucleic acids such as using beta-propriolactone (BPL). Heating at a temperature such as above 85°C, preferably between 85°C and 100°C for suitable period of time, such as above 18 hours and preferably between 18 and 48 hours, even more preferably between 18 and 30 hours may also effective in reducing the viral contaminants. Heating at a lower temperature (84°C, preferably 80°C) may be carried out on solid HA-pancreatin with residual moisture of 0.5 weight % or less.

A method of treatment of a patient subject to a physiological condition associated with pancreatic enzymatic insufficiency is disclosed herein. The method of treatment includes administering to the patient a pharmaceutically acceptable amount of the composition disclosed herein. A method of preparing HA-pancreatin comprising precipitating pancreatin from a solution of native pancreatin with saturated ammonium sulfate is disclosed herein. The method of preparing HA-pancreatin includes the following steps: a) suspending native pancreatin in an aqueous buffer; b)centrifuging the suspension of native pancreatin; c) decanting the supernatant of step b; d) adding an ammonium sulfate solution to the supernatant to form a precipitate; e)centrifuging the suspension of step d to produce a pellet comprising HA-pancreatin, wherein the ammonium sulfate solution is a saturated ammonium sulfate solution and saturated ammonium sulfate is added to the supernatant for a final concentration of about 50-75% saturated ammonium sulfate, preferably at a final concentration of 60% saturated ammonium sulfate. The method further includes washing the pellet with ammonium sulfate, solubilizing the pellet in an aqueous buffer to form a solution of HA-pancreatin., wherein the HA-pancreatin solution is desalted by gel filtration and gel filtration is performed on a column comprising a cross-linked dextran gel. The HA-pancreatin has at least about 50%, 60%, or 70% of the lipase activity of the native pancreatin, a protein concentration of 2-5 times the protein concentration of the native pancreatin and has at least about 60% of the lipase activity, at least about 75% of the amylase activity, and at least about 50% of the protease activity of the native pancreatin. The HA-pancreatin has at least about 60% of the lipase activity, at least about 75% of the amylase activity, and at least about 50% of the protease activity of the native pancreatin and a protein concentration of 2-5 times the protein concentration of the native pancreatin.

It is clear from the above that there are many advantages of the suspending-precipitating approach disclosed in the present disclosure. It allows the preparation of pancreatin with high enzymatic activity. It preserves the digestive enzymes that are present in digestive juices and maintain the digestion behavior of the starting material. It removes those materials that are not required for digestion that remain as a result of the crude extraction process, thus decreasing the bulk of the API. It enables the reduction or removal of bacterial or viral contaminants. It enables a control of the ratios of the mixed enzyme classes to one another. Moreover, it enables the API to be formulated in a number of dosage forms with high potency.

For the preparation of the high activity pancreatin of the invention the suspending-precipitating approach (single-step, two-steps, multi-steps, double precipitation processes) are preferred over the process using the ammonium sulfate precipitation; the double precipitation process is the more preferred process of the invention.

## EXAMPLES

### Materials

Pancrelipase (API, starting pancrelipase or pancreatin, native pancrelipase or pancreatin) is provided by Nordmark, and it is extracted from porcine pancreas. It typically contains about 30% of proteins (quantified with Bradford method) and has a lipase activity of 94.4 IU /mg, protease activity of 253.2 IU /mg, amylase activity of 420.3 IU /mg; P/L ratio is 2.7, A/L ratio is 4.5 and water content is 0.3%.

This material is analyzed by mono and bi-dimensional electrophoresis followed by Maldi Tof characterization in order to identify pancrelipase constituents (Mario Negri Institute, Milan, Italy). Bidimensional electrophoresis on this extract shows there to be at least 50 protein/peptide spots in a fraction that is apparently readily soluble in water and 30 protein/peptide spots in a fraction which is apparently less readily soluble in water. Solubility determinations are confused by the fact that active enzymes may adsorbed onto or otherwise trapped by water-insoluble components of the pancrelipase. Solubility will also be a function of pH. The spots, corresponding to individual proteins in the mixture, and images of gels prepared using the more soluble and the less soluble fractions are analyzed after cutting out and digesting the major spots with bovine trypsin and analysing using matrix-assisted laser desorption/ionization-time of flight mass spectrometry (MALDI-TOF) and zymography. The peptide mass fingerprints are compared with those from library references and amylase, lipase, colipase, carboxypeptidase A1 and B, elastase 2A and 1, chymotrypsin, trypsin, and phospholipase A2 are identified. The extract is clearly both relatively crude and contains many extraneous proteins in addition to a number of active enzyme species.

Enteral formula: PEPTAMEN® Junior 1.0 Cal (Nestle, package of 250 mL): fat content: 3.8 g/100 mL, protein content: 3 g/100mL, carbohydrate content: MLML13.8 g/100 mL.

### Methods

Lipolytic activity: Measurement is carried out with a method based on the compendial procedure of lipase assay described in the pancrelipase enzymes USP monograph, which is based on the titration, by means of pH-stat method, of the free fatty acids formed from the hydrolysis of esterified fatty acids in the substrate used (olive oil). It is based on the following principle: lipase catalyzes the hydrolysis of triglycerides which leads to the formation of free fatty acids (FFA). The titration of the formed FFA according to time provides for the determination of the enzymatic activity of lipase, which can be expressed in units: 1U = 1 $\mu$mole of formed FFA per minute. The reaction occurs by maintaining a steady pH value through an experimental system that provides for the addition of NaOH (titrant) when the pH value changes compared to a fixed value (pHstat method). The quantity of added titrant according to time corresponds to the quantity of FFA formed by the lipase action on the triglycerides. The curve slope {added titrant = f (volume (mL)/ time (minutes))} gives the lipase enzymatic activity.

The lipase activity (LA) reported hereunder is always expressed as IU. The lipase specific activity (LSA) reported hereunder is always expressed as IU /mg.

Proteolytic and amilolytic activity: Measurements are carried out according to the compendial procedure described in the pancrelipase enzymes USP monograph. Specific enzymatic activity (SA) reported hereunder is always expressed as IU /mg.

Water content is measured by TGA at 80°C for 4 hours (samples 18, 19 and 20) or with Karl Fisher method (samples 26, 27 and 28).

Triglycerides are extracted with hexane: isopropanol (3:2) using cholesteryl palmitate as internal standard and analyzed by HPLC. Peaks are identified by comparing all the retention times with a standard triolein solution.

Protein analysis: Total Protein Content is quantified with a Bradford Assay.

Carbohydrate analysis: 1) Short chain sugars are analyzed by HPLC using xylitol as internal standard; peaks are identified by comparing all the retention times with sugar standards i.e., maltose. 2) Maltodextrins are extracted in presence of Carrez I and Carrez II and analyzed by HPLC. Peaks are identified by comparing all the retention times with maltodextrins standards i.e., maltose monohydrate, maltotriose, maltotetraose, maltopentaose, maltohexaose and maltoheptaose.

Saturated Ammonium Sulfate: A saturated ammonium sulfate solution is prepared as follows: saturation is calculated for 4°C and the solution prepared at room temperature, then chilled on ice. For example, 1.43g ammonium sulfate is added to 2 mL of room temperature PBS (50 mM Sodium Phosphate, 150 mM NaCl, pH 7.4) and mixed to dissolve to a final volume of 2.76 mL. After the ammonium sulfate has dissolved, the solution is placed on ice.

Pancreatin Extraction: An extract of 40 mg/mL pancreatin is prepared as follows: powdered pancreatin (about 40-50 mg) is added to a microfuge tube, followed by cold PBS buffer. The tube is shaken to re-suspend the powder and incubated on ice for about 15 minutes with occasional mixing. Alternatively, about 200 mg of powdered pancreatin can be added to a small chilled beaker, followed by 5 mL cold PBS. The mixture is stirred with a magnetic stirrer for about 15-30 minutes on ice and transferred to 1.5 mL microfuge tubes. The microfuge tubes are spun at 16,000g for 5 minutes, either at room temperature or 4°C. The tube is chilled if spun at room temperature, the supernatant decanted, and the extract stored on ice.

Ammonium Sulfate Precipitation: The ammonium sulfate precipitation occurs as follows: in a microfuge tube, 750 $\mu$l of chilled saturated ammonium sulfate is slowly added to 500 $\mu$l of chilled pancreatin extract prepared as discussed above. The mixture is mixed well and incubated on ice for 20 minutes with occasional mixing. The mixture is centrifuged for 5 minutes at 16,000g, and the supernatant decanted and stored on ice.

Pellet wash: The pellet may be washed as follows: the pellet from the ammonium sulfate precipitation is re-suspended in 500 $\mu$l of chilled 60% saturated ammonium sulfate (in PBS buffer). The suspension is incubated on ice for 5 minutes, then centrifuged for 5 minutes at 16,000g, and placed back on ice. The supernatant can then be decanted to leave the washed pellet. The ammonium sulfate pellet may be re-dissolved as follows: 1 mL of chilled PBS is added to the pellet, and then the tube is gently shaken to dissolve. The resulting mixture is incubated on ice for 15 minutes, then centrifuged for 5 minutes at 16,000g to remove any insoluble material. The pellet may also be re-dissolved in 0.5 mL of PBS to make a more concentrated solution.

Desalting: A 4 mL G-25 Sephadex column is equilibrated in deionized water and spun dry at 2000 g for 2 minutes. The sample (0.75mL) to be desalted is added and the column spun at 2000g for 2 minutes.

4-Methylumbelliferyl oleate Assay: A 20 mM stock solution of 4-Methylumbelliferyl oleate n ethanol is prepared and stored at -20°C. A working dilution is prepared on the day of the assay by diluting the ethanol stock with PBS to a concentration of 0.1 mM. The following is mixed: 50 $\mu$l of the 0.1 mM 4-Methylumbelliferyl oleate stock; 25 $\mu$l of PBS; 25 $\mu$L of ~ 0.4 $\mu$g of pancreatin in 50 mM Tris HCl, 100 mM NaCl pH 7.6. Emission at 450 nm with excitation as 355 nm is measured in a plate reader. The assay can be performed as an endpoint assay by adding 1 mL of 5 mM Triton X-100 after about 10 minutes to stop the reaction.

Amylase Assay Protocol: Materials for Amylase Assay: The amylase assay requires: 2,2'-Azino-bis(3-ethylbenzthiozoline-6-sulfonic acid) (ABTS), glucose oxidase, $\alpha$-glucosidase, starch and soybean peroxidase (Rz=1.63). ABTS is stored in 50mM phosphate-citrate pH7 buffer at 4°C. Soybean peroxidase is stored in PBS pH 7.4 at 4°C. Glucose oxdiase is stored in 50mM sodium acetate/100mM NaCl pH5. $\alpha$-Glucosidase and starch are stored in de-ionized water at 4°C. All of the reagents, once brought up as solutions, are kept at 4°C. A 100$\mu$L reaction mixture of 0.1 mg/mL ABTS, 39ug/mL soybean peroxidase (SBP), 10U/mL or 80.6$\mu$g/mL glucose oxidase, 7U/mL or 63$\mu$g/mL glucosidase, and 1mg/mL starch is made at 4°C or on ice. The starch, unlike the other components, is at room temperature before adding to the reaction mix and is also added to the reaction mix immediately before using in the plate. The components of the reaction mix should be added in this order: ABTS, SBP, glucose oxidase, glucosidase, and starch. The reaction is initiated by the addition of 5$\mu$l pancreatin, which is diluted in 1mM PBS/30mM CaCl$_2$, to 95$\mu$l of reaction mix. The final concentration of pancreatin in the assay is 4$\mu$g/mL. The absorbance at 405nm is recorded up to about 15 minutes. The data points are plotted as a function of time. There is a lag phase and the linear part of the curve is usually found between about 8-12 minutes of running the assay. The slopes of the linear portion of the curve are calculated and used as the final data.

Reference Example 1: Multi-steps process

Preparation of HA-pancreatin; 0.1 g/mL; multi-step: suspension, separation, precipitation; (SP= 38: acetone: aqueous solvent = 35: 65; ethanol: aqueous solvent = 45: 55)

Step a1.1- Suspending: Starting pancrelipase is dispersed in aqueous solvent at concentration of 0.1 g/mL at 4°C and kept under stirring for 30 minutes. The experiments are carried out at laboratory scale using either 650 mg (when organic solvent is acetone) or 550 mg (when organic solvent is ethanol) of starting pancrelipase. Four different aqueous solvents are tested for pancrelipase suspension: 1) pH=4.0 buffer (10mM acetate buffer); 2) pH=7.0 buffer (10 mM phosphate); 3) deionized water (DW); 4) pH=4.0 buffer (10mM acetate) with NaCl (0.5M).

Step a1.2- Separating: The suspension from step a1.1 is centrifuged (10 minutes, 4°C, about 11,000 g) and the supernatant (SN) is separated from the pellet (P).

Step a1.3- Precipitating: The organic solvent is added to the supernatant of step a1.2 and the mixture is kept at 4°C for 15 minutes in static condition. The organic solvent is either acetone or ethanol. Acetone is added in amount of 35 parts (volume) per each 65 parts (volume) of aqueous solvent. Ethanol is added in amount of 45 parts (volume) per each 55 parts (volumes) of aqueous solvent.

Step a2- Separating: The mixture is centrifuged (10 minutes, 4°C, about 11,000 g) to separate supernatant from pellet, which contains the pancrelipase enzymes. The pellet is re-suspended in the aqueous solvent used in step a1.1 (LA in pellets after re-suspension, precipitation).

The materials of the different steps are analyzed. The amount of pancrelipase, expressed as lipase activity (LA) is measured:

-   in suspension of step a1.1 (LA in suspension; LA-Sa1.1);

-   in supernatant obtained in step a2 (LA in SN, precipitation, LA-SN);

-   in pellets obtained in step a2 and then re-suspended in the starting medium (LA in pellets, precipitation, LA-P).

Results are reported in Tables 1, 2, 3, 4.

Table 1

| Sample | Experimental conditions | | Suspension (Step a1.1) | Separation (Step a2) | | | | Yield (%) |
| | Medium | Solvent | LA-Sa1.1 | Supernatant (SN) | | Pellet (P) | | |
| | | | | LA-SN | % LA-SN/ LA-Sa1.1 | LA-P | % LA-P/ LA-Sa1.1 | |
| 1 | pH 4.0 | A | 33528.2 | 5820.0 | 17.4 | 25376.4 | 75.7 | 93.1 |
| 2 | Water | A | 31974.0 | 8105.5 | 25.4 | 22659.2 | 70.9 | 96.3 |
| 3 | pH 4.0 | E | 35388.9 | 3464.3 | 9.8 | 27417.6 | 77.5 | 87.3 |
| 4 | Water | E | 30369.8 | 4301.0 | 14.2 | 20835.1 | 68.6 | 82.8 |
| 5 | pH 4.0+ NaCl | A | 47761.0 | 9408.0 | 19.7 | 34543.3 | 72.3 | 92.0 |
| 6 | pH 4.0+ NaCl | E | 40364.0 | 10410.4 | 25.8 | 27101.5 | 67.1 | 92.9 |
| 7 | pH 7.0 | A | 40388.9 | 9798.8 | 24.3 | 30178.8 | 74.7 | 99.0 |
| LA=lipase activity (IU); A= acetone. E=ethanol; S=suspension; SN=supernatant; P=pellet | | | | | | | | |

Table 1 shows that precipitation of pancrelipase (step a1.3) allows for a good recovery of lipase activity in the precipitate portion (pellet), which ranges from about 67 to about 77 %. Yield is the % of total lipase activity of step a2 (pellet and supernatant) with respect to the lipase activity of the initial suspended pancrelipase, expressed as lipase activity in the suspension of step a1.1 (Sa1.1): (LA-SN +LA-P)/(LA-Sa1.1).

Table 2

| Sample | Experimental conditions | | Theor. LA in Step a1.1 | Separation (Step a2) | | | | Yield (%) |
| | Medium | Solvent | | Supernatant (SN) | | Pellet (P) | | |
| | | | | LA-SN | % LA-SN/ theor LA-Sa1.1 | LA-P | % LA-P / theor LA-Sa1.1 | |
|---|---|---|---|---|---|---|---|---|
| 1 | pH 4.0 | A | 61605.4 | 5820.0 | 9.4 | 25376.4 | 41.2 | 50.6 |
| 2 | Water | A | 61458.2 | 8105.5 | 13.2 | 22659.2 | 36.9 | 50.1 |
| 3 | pH 4.0 | E | 51940.8 | 3464.3 | 6.7 | 27417.6 | 52.8 | 59.5 |
| 4 | Water | E | 51894.0 | 4301.0 | 8.3 | 20835.1 | 40.1 | 48.4 |
| 5 | pH 4.0+ NaCl | A | 61452.0 | 9408.0 | 15.3 | 34543.3 | 56.2 | 71.5 |
| 6 | pH 4.0+ NaCl | E | 52031.5 | 10410.4 | 20.0 | 27101.5 | 52.1 | 72.1 |
| 7 | pH 7.0 | A | 61470.4 | 9798.8 | 15.9 | 30178.8 | 49.1 | 65.0 |
| LA=lipase activity (IU); A= acetone. E=ethanol; S=suspension; SN=supernatant; P=pellet; Theor.=theoretical. | | | | | | | | |

The process yield ranges from about 37 to about 56%. Yield % is the total lipase activity in the pellet and in supernatant of step a2 with respect to theoretical lipase activity of pancrelipase used in step a1.1, which is calculated by factoring specific activity of untreated raw material (i.e., 94.4 IU /mg as determined according to compendial USP method) and its initial weight.

Table 3

| Sample | Experimental conditions | | Separation (Step a2) | | EF |
| | Medium | Solvent | Supernatant (SN) | Pellet (P) | |
| | | | LSA | LSA | |
|---|---|---|---|---|---|
| 1 | pH 4.0 | A | 10.7 | 239.4 | 2.5 |
| 2 | Water | A | 14.5 | 233.6 | 2.5 |
| 3 | pH 4.0 | E | 10.1 | 179.2 | 1.9 |
| 4 | Water | E | 11.5 | 145.7 | 1.5 |
| 5 | pH 4.0+ NaCl | A | 14.7 | 101.3 | 1.1 |
| 6 | pH 4.0+ NaCl | E | 18.2 | 80.9 | 0.9 |
| 7 | pH 7.0 | A | 18.7 | 181.8 | 1.9 |
| LSA= lipase specific activity (IU /mg); A= acetone. E=ethanol; SN=supernatant; EF= enrichment factor= LSA in pellet of step a2/ theoretical lipase specific activity in step 1.1a, *i.e.*, 94.4 IU /mg as determined according to compendial USP method. | | | | | |

The enrichment factor (pH independent) is determined by calculating the ratio between the lipase activity in the pellet of step a2 and the lipase activity of the starting pancreatin (which is 94.4 IU/mg). Enrichment factor up to 2.5 is obtained with this process; the enrichment is confirmed also by HPLC profile analysis.
With regards to the other digestive enzymes, the amylase content in the final pellet (precipitate of step a2) is lower than the amylase content in the starting pancrelipase.

Reference Example 2: Multi-step process

Preparation of HA-pancreatin; 0.3 g/mL; multi-step: suspension, separation, precipitation (SP= 38: acetone: aqueous solvent = 35: 65; ethanol: aqueous solvent = 45: 55)

Step a1.1- Suspending: Pancrelipase (API) is dispersed in aqueous solvent at concentration of 0.3 g/mL at 4°C and stirred for 30 minutes. The experiment is carried out at laboratory scale using either 0.650 ml (when organic solvent is acetone) or 0.550 ml (when organic solvent is ethanol) of starting pancrelipase. Two experiments are run each in a different aqueous solvent: 1) pH=4.0 buffer (10mM acetate buffer); 2) pH=7.0 buffer (10 mM phosphate).

Step a1.2- Separating: The suspension from step a1 is centrifuged (10 minutes, 4°C, about 11,000 g) and the supernatant (SN) is separated from the pellet (P).

Step a1.3- Precipitating: The organic solvent is added to the supernatant of step a1.2 and the mixture is kept at 4°C for 15 minutes in static condition. The organic solvent is either acetone or ethanol. Acetone is added in amount of 35 parts (volume) per each 65 parts (volume) of aqueous solvent. Ethanol is added in amount of 45 parts (volume) per each 55 parts (volumes) of aqueous solvent.

Step a2- Separating: The mixture is centrifuged (10 minutes, 4°C, about 11,000 g) to separate supernatant from pellet, which contains the pancrelipase enzymes. The pellet is re-suspended in the starting aqueous solvent (LA in pellets after re-suspension, precipitation).

Step a3- Drying: The pellet of step a2 is dried.

The materials of the different steps are analyzed. The amount of pancrelipase is expressed as lipase activity (LA) and it is measured:

- in suspension of step a1.1 (LA in suspension; LA-Sa1.1);

- in supernatant obtained in step a2 (LA in SN, precipitation, LA-SN);

- in pellets obtained in step a2 and then re-suspended in the starting aqueous medium (LA in pellets, precipitation, LA-P). Results are reported in Tables 4, 5, 6.

Table 4

| Sample | Experimental conditions | | Suspension (Step a1.1) | Separation (Step a2) | | | | Yield (%) |
|---|---|---|---|---|---|---|---|---|
| | | | | Supernatant (SN) | | Pellet (P) | | |
| | Medium | Solvent | LA (Sa1.1) | LA-SN | % LA-SN/ LA-Sa1.1 | LA-P | % of LA-P/ LA-Sa1.1 | |
| 8 | pH 4.0 | A | 139968.7 | 11856.6 | 8.5 | 112830.4 | 80.6 | 89.1 |
| 9 | pH 4.0 | E | 107348.7 | 5035.2 | 4.7 | 99146.6 | 92.4 | 97.0 |
| 10 | pH 4.0 | A | 137050.4 | 8778.9 | 6.4 | 108734.7 | 79.3 | 85.7 |
| 11 | pH 4.0 | E | 115965.7 | 5097.9 | 4.4 | 88019.7 | 75.9 | 80.3 |
| 12 | pH 7.0 | A | 137267.4 | 10535.3 | 7.7 | 108038.6 | 78.7 | 86.4 |
| 13 | pH 7.0 | E | 118929.3 | 11340.9 | 9.5 | 86636.2 | 72.8 | 82.4 |
| LA=lipase activity (IU); ; A= acetone. E=ethanol; S=suspension; SN=supernatant; P=pellet | | | | | | | | |

Table 4 shows that recovery after precipitation is very good (73-92%).

Table 5

| Sample | Experimental conditions | | | Separation (Step a2) | | | | Yield (%) |
| | Medium | Solvent | Theor. LA in Step a1.1 | Supernatant (SN) | | Pellet (P) | | |
| | | | | LA | % LA-SN/ theor LA-Sa1.1 | LA | % LA-P/ theor LA-Sa1.1 | |
|---|---|---|---|---|---|---|---|---|
| 8 | pH 4.0 | A | 184080.0 | 11856.6 | 6.4 | 112830.4 | 61.3 | 67.7 |
| 90 | pH 4.0 | E | 155760.0 | 5035.2 | 3.2 | 99146.6 | 63.7 | 66.9 |
| 10 | pH 4.0 | A | 184080.0 | 8778.9 | 4.8 | 108734.7 | 59.1 | 63.8 |
| 11 | pH 4.0 | E | 155760.0 | 5097.9 | 3.3 | 88019.7 | 56.5 | 59.8 |
| 12 | pH 7.0 | A | 184080.0 | 10535.3 | 5.7 | 108038.6 | 58.7 | 64.4 |
| 13 | pH 7.0 | E | 155760.0 | 11340.9 | 7.3 | 86636.2 | 55.6 | 62.9 |
| LA=lipase activity (IU); ; A= acetone. E=ethanol; S=suspension; SN=supernatant; P=pellet; Theor.=theoretical. | | | | | | | | |

The yield for the pellet of multi-steps process carried out on 0.3g/mL pancrelipase concentration ranges from about 56 to about 64.

Table 6

| Sample | Experimental conditions | | Separation (Step a2) | | EF |
| | Medium | Solvent | Supernatant (SN) | Pellet (P) | |
| | | | LSA | LSA | |
|---|---|---|---|---|---|
| 8 | pH 4.0 | A | 6.2 | 197.9 | 2.1 |
| 9 | pH 4.0 | E | 5.3 | 250.4 | 2.7 |
| 10 | pH 4.0 | A | 7.5 | 284.6 | 3.0 |
| 11 | pH 4.0 | E | 5.5 | 185.7 | 2.0 |
| 12 | pH 7.0 | A | 8.4 | 248.9 | 2.6 |
| 13 | pH 7.0 | E | 11.1 | 180.9 | 1.9 |
| LSA= lipase specific activity (IU /mg) ; A= acetone. E=ethanol; SN=supernatant; EF= enrichment factor= LSA in pellet step a2 (IU /mg)/ theoretical lipase activity in step a1.1 (IU /mg). | | | | | |

The enrichment factor is determined by calculating the ratio between the lipase activity in the pellet of step a2 and the lipase activity of the starting pancrelipase of step a1.1 (which is 94.4 U/mg). Enrichment factor ranges from 1.9 to 3.0.

Reference Example 3: Two-steps process

Preparation of HA-pancreatin; 0.1 g/mL; two-step: suspension, precipitation (SP= 38: acetone: aqueous solvent = 35: 65; ethanol: aqueous solvent = 45: 55, where SP (acetone)=20.2, SP(ethanol)= 26.0, SP (buffer) =47.9)

Step a1.1- Suspending: Pancrelipase is dispersed in aqueous solvent at concentration of 0.1 g/mL at 4°C and kept under stirring for 30 minutes. The experiment is carried out at laboratory scale using either 650 mg (when organic solvent is acetone) or 550 mg (when organic solvent is ethanol) of starting pancrelipase. Two experiments are run, each in a different aqueous solvent: 1) pH=4.0, 10 mM acetate buffer; 2) pH=7.0, 10 mM phosphate buffer.

Step a1.2- Precipitating: The organic solvent is added to the suspension of step a1.1 and this mixture is kept at 4°C for 15 minutes. The organic solvent is either acetone or ethanol. Acetone is added in amount of 35 parts (volume) per each 65 parts (volume) of aqueous solvent. Ethanol is added in amount of 45 parts (volume) per each 55 parts (volumes) of aqueous solvent.

Step a2- Separating: The mixture is centrifuged (10 min, 4°C, about 11,000 g) to separate supernatant from pellet, which contains the pancreatic enzymes. The pellet is re-suspended in the starting aqueous solvent (LA in pellets after re-suspension, separation).

The materials of the different steps are analyzed. The amount of pancrelipase, expressed as lipase activity, is measured along the whole process:

- in suspension of step a1.1 (LSA in suspension; LA-Sa1.1);

- in supernatant obtained in step a2 (LA in SN, separation, LA-SN);

- in pellets obtained in step a2 and then re-suspended in the starting medium (LA in pellets, precipitation, LA-P).

Results are reported in Tables 7, 8, 9.

Table 7

| Sample | Experimental conditions | | Suspension (Step a1.1) | Separation (Step a2) | | | | Yield (%) |
|---|---|---|---|---|---|---|---|---|
| | | | | Supernatant (SN) | | Pellet (P) | | |
| | Medium | Solvent | LA (Sa1.1) | LA | % LA-SN/ LA-Sa1.1 | LA | % LA-P/ LA-Sa1.1 | |
| 14 | pH 4.0 | A | 48960.7 | 4688.0 | 9.6 | 45021.4 | 92.0 | 101.5 |
| 15 | pH 4.0 | E | 41428.3 | 1394.3 | 3.4 | 40656.7 | 98.1 | 101.5 |
| 16 | pH 7.0 | A | 56936.9 | 4750.9 | 8.3 | 46955.0 | 82.5 | 90.8 |
| 17 | pH 7.0 | E | 48177.4 | 2316.1 | 4.8 | 41808.2 | 86.8 | 91.6 |
| LA=lipase activity (IU); A= acetone. E=ethanol; S=suspension; SN=supernatant; P=pellet. | | | | | | | | |

Recovery after precipitation is high, almost complete recovery is achieved with the two-steps process.

Table 8

| Sample | Experimental conditions | | Separation (Step a2) | | | | | Yield (%) |
|---|---|---|---|---|---|---|---|---|
| | | | Theor. LA Step1a | Supernatant (SN) | | Pellet (P) | | |
| | Medium | Solvent | | LA | %LA-SN/theor SL-S1a | LA | % LA-P/ theor LA-S1a | |
| 14 | pH 4.0 | A | 61605.4 | 4688.0 | 7.6 | 45021.4 | 73.1 | 80.7 |
| 15 | pH 4.0 | E | 51940.8 | 1394.3 | 2.7 | 40656.7 | 78.3 | 81.0 |
| 16 | pH 7.0 | A | 61421.4 | 4750.9 | 7.7 | 46955.0 | 76.4 | 84.2 |
| 17 | pH 7.0 | E | 51971.9 | 2316.1 | 4.5 | 41808.2 | 80.4 | 84.9 |
| LA=lipase activity (IU); ; A= acetone. E=ethanol; S=suspension; SN=supernatant; P=pellet; Theor.= theoretical. | | | | | | | | |

The process yield for the pellet ranges from 73.1 to 80.4, which is higher than that obtained with the multi-step process. The yield % is the total lipase activity in the pellet and in supernatant of step a2 with respect to theoretical lipase activity of pancreatin used in step a1.1, which is calculated by factoring specific activity of starting material (*i.e.,* 94.4 IU /mg as determined according to compendial USP method) and its initial weight.

Table 9

| Sample | Experimental conditions | | Separation (Step a2) | | EF |
| | Medium | Solvent | Supernatant (SN) | Pellet (P) | |
| | | | LSA | LSA | |
| 14 | pH 4.0 | A | 10.1 | 247.4 | 2.6 |
| 15 | pH 4.0 | E | 4.0 | 204.3 | 2.2 |
| 16 | pH7.0 | A | 10.1 | 276.2 | 2.9 |
| 17 | pH7.0 | E | 6.7 | 193.6 | 2.1 |
| LSA= lipase specific activity (IU /mg) ; A= acetone. E=ethanol; SN=supernatant; EF= enrichment factor = LSA in pellet step a2 (IU /mg)/ theoretical lipase activity in step a1.1 (IU /mg). | | | | | |

The results show that the use of the aqueous buffer at pH=7 and acetone in two-step process provide interesting yield (process yield for the pellet of about 76%) and enrichment factor ranges from 2.1 to 2.9.

Reference Example 4: Two-steps process

Preparation of HA-pancreatin; 0.1 g/mL; two- steps: suspension, precipitation; pilot (SP= 38: acetone: aqueous solvent=35:65)

Step a1.1- Suspending: 6.5 g of starting pancrelipase (61,400 U lipase) is dispersed in 45 mL of pH=7.0 buffer solution (10 mM phosphate buffer) at 4°C and stirred (Ultraturrax, 3 cycles) for 1 minute for each cycle, stirrer is washed two times with 10 mL of cold buffer in order to recover the residual pancrelipase.

Step a1.2- Precipitating: 35 mL of acetone is added to the suspension of step a1.1 and the mixture is kept at 4°C for 30 minutes under static condition.

Step a2- Separating: The mixture is centrifuged (10 minutes, 4°C, about 2,700 g) to separate supernatant from pellet, which contains the pancreatic enzymes.

Steps a3- Drying: The pellet is dried according to two different protocols the mean weighted dried pellet is 1.55 g, the lipase activity (LA) is 365,000 IU lipase, the specific activity (LSA) is 235 IU /mg.

Table 10 reports lipase (L), protease (P), and amylase (A) specific activities measured for each material obtained with the two protocols. Lipase activity in the pellets (measured after its suspension in 1 mL of buffer) is measured also before the drying treatment; it amounts to 233.7 IU /mg.

Table 10

| Sample | Protocols | LSA | PSA | ASA | P/L ratio | A/L ratio | Water content (%) |
|---|---|---|---|---|---|---|---|
| 18 | 72 h 6-80° C 0.2 mbar | 234.2 | 226.5 | 149.5 | 0.97 | 0.64 | 7.4 |
| 19 | 72 h 6-8° C 0.2 mbar | 254.0 | 217.7 | 130.7 | 0.86 | 0.51 | 6.5 |
| 20 | 24 h 6-8° C 0.2 mbar | 216.3 | 230.1 | 129.8 | 1.06 | 0.60 | 7.4 |
| Mean | | 234.8 | 224.8 | 136.7 | 0.96 | 0.58 | 7.10 |
| Ds | | 18.9 | 6.4 | 11.1 | | | |
| cv% | | 8% | 3% | 8% | | | |
| LSA= lipase specific activity (IU /mg); PSA= protease specific activity (IU /mg); ASA = amylase specific activity (IU /mg). | | | | | | | |

The analysis shows that drying process itself does not affect LSA and that different protocols leads to materials with same enzymatic activities. The enrichment factor calculated as in previous examples is also always above 2 and is constant among the different protocols. It is: 2.5 (sample 18), 2.7 (sample 19), 2.3 (sample 20), mean value is 2.5. Lipase activity in the pellets, measured before the drying treatment, amounts to 233.7 IU /mg (sample 18).

Table 11

| Sample | W starting pan | W pellet | Initial LA | Initial PA | Initial AA | Recovered LA | Recovered PA | Recovered AA | LY | P Y | AY |
|---|---|---|---|---|---|---|---|---|---|---|---|
| 18 | 6.5113 | 1.5325 | 614666.7 | 1648661.2 | 2736699.4 | 358911.5 | 347111.3 | 229108.8 | 58 | 21 | 8 |
| 20 | 6.4997 | 1.7084 | 613571.7 | 1645724.0 | 2731823.9 | 369526.9 | 393102.8 | 221750.3 | 60 | 24 | 8 |
| 19 | 6.5137 | 1.3673 | 614893.3 | 1649268.8 | 2737708.1 | 347294.2 | 297661.2 | 178706.1 | 56 | 18 | 7 |
| L= lipase; P= protease; A= amylase; A= activity; Y=yield (%); W =weight (g), for pellet measured after drying. | | | | | | | | | | | |

The HPLC profiles of samples obtained with the different protocols are super-imposable; no relevant qualitative difference with the HPLC profile of the starting material was observed.

Reference Example 5: Two-step process

Preparation of HA-pancreatin; 0.1 g/mL; two-step: suspension, precipitation; scale-up (SP= 38: ethanol: aqueous solvent (pH=7) = 45: 55, sample 21); (SP= 34: acetone: aqueous solvent (pH=7) = 50: 50 sample 22); (SP= 35: acetone: aqueous solvent (pH=7) = 45: 55, sample 23). The preparation process of Example 4 is applied on different amount of starting pancrelipase, different volume of buffer solution and different volume of acetone (SP=34 or 35) or volume of ethanol (SP=38). The drying protocol is 24 hours, 6-8°C, 0.2 mbar, all other parameters and conditions are the same as in Example 4.

Table 12

| Sample | HS | Pancrelipase (g) | Buffer (mL) | Acetone (mL) | Ethanol (mL) |
|---|---|---|---|---|---|
| 21 | 38 | 5.5 | 55 | no | 45 |
| 22 | 34 | 5.0 | 50 | 50 | no |
| 23 | 35 | 5.5 | 55 | 45 | no |

Results are reported in Tables 13, 14.

Table 13

| Sample | LSA | PSA | ASA | P/L ratio | A/L ratio |
|---|---|---|---|---|---|
| 21 | 150.2 | - | - | - | - |
| 22 | 123.3 | 351.7 | 436.0 | 2.85 | 3.54 |
| 23 | 158.7 | 388.9 | 241.9 | 2.45 | 1.52 |
| LSA= lipase specific activity(IU /mg); PSA= protease specific activity(IU /mg); ASA= amylase specific activity (IU /mg). | | | | | |

The enrichment factor calculated as in previous Examples is: 1.6 (sample 21), 1.3 (sample 22), 1.7 (sample 23).
Table 14 shows the enzymatic activities of the final purified material obtained with the scaled-up process here applied and the enzymatic yield.

Table 14

| Sample | W starting pan | W of pellet | Initial LA | Initial PA | Initial AA | Recovered LA | Recovered PA | Recovered AA | LY | P Y | AY |
|---|---|---|---|---|---|---|---|---|---|---|---|
| 21 | 5.5197 | 1.6826 | 514988.0 | - | - | 252390.0 | - | - | 49 | - | - |
| 22 | 5.5183 | 2.0255 | 520927.5 | 1397233.6 | 2319341.5 | 321446.9 | 787717.0 | 489968.5 | 62 | 56 | 21 |
| 23 | 4.9986 | 2.7464 | 471867.8 | 1265645.5 | 2100911.6 | 338631.1 | 965908.9 | 1197430.4 | 72 | 76 | 57 |
| L= lipase; P= protease; A= amylase; A= activity; Y=yield (%);W =weight (g), for pellet measured after drying. | | | | | | | | | | | |

No relevant qualitative difference with the HPLC profile of the starting material wassobserved.

Samples 22 and 23 obtained with different protocol show a lower specific LA and higher PA and AA over the sample 20 produced with protocol (SP=38).

Reference Example 6: Single-step process

Preparation of HA-pancreatin; 0.065 and 0.1 g/mL; single-step; lab scale (SP= 38-acetone: aqueous solvent = 35: 65)

Step a1- Suspending- precipitation: 650 mg (sample 24) or 1000 mg (sample 25) of native pancrelipase are dispersed in 10 mL of a 65:35 mixture of buffer (pH=7 10 mM phosphate) and acetone (65 volumes of buffer and 35 volumes of acetone) under stirring for 60 minutes at 4°C.

Step a2- Separating: The mixture of step a1 is centrifuged (10 min at 10,000 g at 4 °C) to separate supernatant from pellet, which contains the pancreatic enzymes.

Step a3 -Drying: The pellet is dried with an high efficiency pump at 0.2 mbar.

The material is analyzed. The amount of pancrelipase, expressed as lipase activity is measured along the whole process:
- in supernatant obtained in step a2 (LA in SN, separation, LA-SN);
- in pellets obtained in step a2 and then re-suspended in the starting medium (LA in pellets, precipitation, LA-P);
- the LA of the suspension - precipitation step a1 (LA in suspension; LA-SN) Results are reported in Tables 15 and 16.

Table 15

| Sample | Experimental conditions | | | Separation (Step a2) | | | | |
|---|---|---|---|---|---|---|---|---|
| | Medium | Solvent | Theor. Initial LA Step a1 | Supernatant (SN) | | Pellet (P) | | Yield (%) |
| | | | | LA | % LA-SN/ theor SL-Sa1 | LA | % LA-P/ theor LA-Sa1 | |
| 24 | pH7.0 | A | - | 2834.7 | - | 47807.9 | - | - |
| 25 | pH7.0 | A | - | 3360.4 | - | 71997.8 | - | - |
| LA=lipase activity (IU); A= acetone; E=ethanol; S=suspension; SN=supernatant; P=pellet; Theor.= theoretical. | | | | | | | | |

Table 16

| Sample | Experimental conditions | | Separation (Step a2) | | EF |
|---|---|---|---|---|---|
| | Medium | Solvent | Supernatant (SN) | Pellet (P) | |
| | | | LSA | LSA | |
| 24 | pH 7.0 | A | 5.4 | 237.9 | 2.5 |
| 25 | pH 7.0 | A | 5.7 | 265.7 | 2.8 |
| LA= lipase activity; A= acetone. E=ethanol; SN=supernatant; EF= enrichment factor = LSA in pellet step a2 (IU /mg) / l lipase specific activity in native raw material(IU /mg). | | | | | |

The single-step process provided good yield and enhancement factor. This is useful for industrialization since the execution is easy and straightforward.

Table 16a reports lipase specific activity (LSA), for purified material obtained with different suspension - precipitation time.

Table 16a. Lipase activity at different times

| Time (min) | LSA | |
|---|---|---|
| | Supernatant (SN) | Pellet (P) |
| 15 | 5.3 | 238.6 |
| 30 | 3.7 | 233.8 |
| 45 | 3.6 | 244.6 |
| 60 | 4.4 | 264.3 |
| LSA= lipase specific activity(IU /mg); SN=supernatant; P=pellet. | | |

Table 16b. Lipase specific activity, comparison of different methods.

| Sample | Method | LSA | |
|---|---|---|---|
| | | Supernatant (SN) | Pellet (P) |
| 16 | Two-steps process | 10.1 | 276.2 |
| 24 | Single-step Process | 5.4 | 237.9 |
| 25 | Single-step Process | 5.7 | 265.7 |
| LSA= lipase specific activity(IU /mg); SN=supernatant; P=pellet. | | | |

The data showed that method used by itself does not affect lipase partitioning between the phases.

Reference Example 7: Single-step process

Preparation of HA-pancreatin; 0.1 g/mL; single-step; pilot scale (SP= 38- acetone: aqueous solvent = 35: 65).

Step a1- Suspending- precipitation: 10 g of native pancrelipase are dispersed in 100 mL of a solvent mixture of buffer (pH=7, 10 mM phosphate) and acetone (65 volumes of buffer and 35 volumes of acetone) under stirring for 60 minutes at 4°C.

Step a2- Separating: The mixture of step a1 is centrifuged (10 min at 2,700 g at 4°C) to separate supernatant from pellet, which contains the pancreatic enzymes.

Step a3- Drying: The pellets of sample 26 is poured into petri dishes, whereas pellet of samples 27 and 28 are kept in the centrifugation tubes and directly dried using a high efficiency pump at 0.2 mbar.

The material is analyzed. The amount of pancrelipase, expressed as lipase activity is measured in:

- in pellets obtained in step a2 and dried according step a3.-

Results are reported in Tables 17 and 18.

Table 17

| Sample | LSA | PSA | ASA | P/L ratio | A/L ratio |
|---|---|---|---|---|---|
| 26 | 207.8 | 249.4 | 155.2 | 1.20 | 0.75 |
| 27 | 222.7 | 249.2 | 158.9 | 1.12 | 0.71 |
| 28 | 219.1 | 285.0 | 167.5 | 1.30 | 0.76 |
| LSA= lipase specific activity (IU /mg); PSA = Protease specific activity (IU /mg); ASA = Amylase specific activity (IU /mg). | | | | | |

Good reproducibility in term of lipase, protease, amylase activity was obtained.

Table 18

| Sample | W starting pan | Wof pellet | LY | PY | AY |
|---|---|---|---|---|---|
| 26 | 10.02 | 3.94 | 87 | 39 | 15 |
| 27 | 10.01 | 4.22 | 100 | 42 | 16 |
| 28 | 10.02 | 4.28 | 99 | 48 | 17 |
| L= lipase; P= protease; A= amylase; A= activity; Y=yield (%); W =weight (g), for pellet measured after drying. |||||||

Very high lipase yield was obtained, and enrichment factor was high (above 2). For example, the enrichment factor was 2.2 (Sample 26), 2.4 (Sample 27), 2.3 (Sample 28).

The single-step process provided good yields. 4.2 g of HA-pancreatin is obtained (42% of the starting pancrelipase), total units of lipase is 940,000 IU (99-100% of the initial LA). The obtained HA-pancreatin has specific activity of 221 IU /mg considering the average between 27 and 28.

The HPLC, SDS-Page profiles and UV spectra of samples 26-28 obtained with the different protocols were super-imposable. No relevant qualitative difference with the HPLC profile of the starting material was observed.

Table 18a

| Sample | Scale | Method | LSA | |
|---|---|---|---|---|
|  |  |  | Supernatant (SN) | Pellet (P) |
| 25 | Lab scale | Single-step Process | 5.7 | 265.7 |
| 29 | Pilot scale | Single-step Process | 6.2 | 250.1 |
| LSA= lipase specific activity(IU /mg); SN=supernatant; P=pellet. |||||

The data show in table 18a that scale does not affect lipase partitioning between the phases and process results scalable accordingly.

Reproducibility of the single-step process was assessed using different Pancrelipase Raw Material listed in Table 18b.

Table 18b. Pancreatin raw material

| Raw Material Code | Starting LSA (IU /mg) |
|---|---|
| 005 | 94.4 |
| 005A | 81.1 |
| 005B | 85.5 |
| 004 | 70.4 |
| 008 | 99.6 |
| LSA= lipase specific activity (IU /mg). ||

Table 18c. Single-step process reproducibility

|  | Starting raw material code | | | | | | | | | |
|---|---|---|---|---|---|---|---|---|---|---|
|  | 005 | | 005A | | 005B | | 004 | | 008 | |
|  | Native | Sample 27 | Native | Sample 30 | Native | Sample 31 | Native | Sample 32 | Native | Sample 33 |
| LSA | 94.4 | 222.7 | 81.1 | 168.1 | 85.5 | 184.3 | 70.4 | 145.9 | 99.6 | 227.2 |
| EF | NA | 2.4 | NA | 2.1 | NA | 2.2 | NA | 2.1 | NA | 2.3 |

(continued)

| | Starting raw material code | | | | | | | | | |
|---|---|---|---|---|---|---|---|---|---|---|
| | 005 | | 005A | | 005B | | 004 | | 008 | |
| | Native | Sample 27 | Native | Sample 30 | Native | Sample 31 | Native | Sample 32 | Native | Sample 33 |
| LY | NA | 99.5 | NA | 89.1 | NA | 88.4 | NA | 82.9 | NA | 82.1 |
| LSA= lipase specific activity(IU /mg); EF= enrichment factor = LSA in pellet step a2 (IU /mg)/lipase activity in native raw material (IU /mg); LY = Lipase Yield (%). | | | | | | | | | | |

The data in Table 18c show that native raw material with different starting LSA does not affect purification process outcome in terms of enhancement factor and good yields were obtained.

Reference Example 8: Comparative example

Preparation of HA-pancreatin; 0.1 g/mL; suspension, precipitation, separation; comparative example (SP= 38-acetone: water = 35: 65).

Step 1- Suspending: Pancrelipase is dispersed in aqueous solvent (distilled water at concentration of 0.045 g/mL at 4°C (sample 29), for about 30 minutes under stirring.

Step 2- Precipitating: 80 mL of the solvent mixture (acetone: water = 35:45) is added to 20 mL of suspension of step a1.1 (to obtain SP=38 (MPa)$^{0.5}$ in the final solvent mixture). The mixture is kept at 25°C for 60 minutes under static condition.

Step 3- Separating: The mixture is centrifuged (10 min at 3,000 g at 25°C) to separate supernatant from pellet, which contains the pancreatic enzymes.

The materials of the different steps are analyzed. The amount of pancrelipase, expressed as lipase activity is measured along the whole process:

- in suspension of step 1 (LA in suspension; LA-Sa1);

- in supernatant obtained in step 2 (LA in SN, precipitation, LA-SN);

- in pellets obtained in step 3 and then re-suspended in the starting distilled water (LA in pellets, precipitation, LA-P).

Results are reported in Tables 19-20. Results obtained with two-steps and one-step methods in previous Examples are also reported here for direct comparison purposes.

Table 19

| Sample | Experimental conditions | | | Separation (Step 2) | | | | Yield (%) |
|---|---|---|---|---|---|---|---|---|
| | Medium | Solvent | Initial LA Step 1 | Supernatant (SN) | | Pellet (P) | | |
| | | | | LA | % LA-SN/ SL-Sa1 | LA | % LA-P/ LA-Sa1 | |
| 16 | pH 7.0 | A | 56936.9 | 4750.9 | 8.3 | 46955.0 | 82.5 | 90.8 |
| 18 | pH 7.0 | A | - | 2834.7 | - | 47807.9 | - | - |
| 19 | pH 7.0 | A | - | 3360.4 | - | 71997.8 | - | - |
| 29 | Water | A | 66313.9 | 7928.8 | 12.0 | 24082.9 | 36.3 | 48.3 |
| LA=lipase activity (IU); A= acetone, E=ethanol; S=suspension; SN=supernatant; P=pellet; | | | | | | | | |

Table 20

| Sample | Experimental conditions | | Precipitation (Step a2) | | EF |
| | Medium | Solvent | Supernatant (SN) | Pellet (P) | |
| | | | LSA | LSA | |
| --- | --- | --- | --- | --- | --- |
| 16 | pH 7.0 | A | 10.1 | 276.2 | 2.9 |
| 18 | pH 7.1 | A | 5.4 | 237.9 | 2.5 |
| 19 | pH 7.0 | A | 5.7 | 265.7 | 2.8 |
| 29 | Water | A | 9.7 | 27.1 | 0.3 |
| LSA= lipase specific activity (IU /mg); A= acetone. E=ethanol; SN=supernatant; EF= enrichment factor = LSA in pellet step a2 (IU /mg)/ lipase specific activity of native raw material (IU /mg). | | | | | |

This comparative process provided poor yield, enzymatic inactivation is pronounced and no lipase enrichment was obtained.

Reference Example 9 Characterization of purified HA-pancreatin (Sample 20)

The HA material is tested for its digestion capability and compared with starting pancrelipase. 20 mg of HA-pancreatin and 50 mg of starting pancrelipase (corresponding to 2300 IU lipase) are suspended each in 1 mL of deionized water and the added to 50 mL of enteral formula PEPTAMEN® Junior 1.0 at 37°C and stirred at 100 rpm for 60, 120 and 240 minutes. Digestion tests were carried out after 1, 2 and 4 hours. The test is repeated 6 times for each pancrelipase sample.

Lipidic nutrients are monitored by measuring the digestion of triolein (decrease of triolein peak); the total protein digestion is monitored by the Bradford method. Amyliolitic process is monitored by measuring the formation of short chain sugars. Digestion extent difference is obtained by comparing the digestion performance of native and HA materials after 4 hours digestion.

Table 21

| Sample | W | LA | PA | AA |
| --- | --- | --- | --- | --- |
| N | 50 | 4,720 | 12,660 | 21,015 |
| H | 20 | 4,142 | 4,354 | 2,614 |
| Activity difference (%) | | -12 | -66 | -88 |
| Digestion difference (%) | | -10 | 6 | -33 |
| H= HA-pancreatin; N= native pancreatin; W= weight (mg) | | | | |

Activity difference is calculated with the formula:

$$(\text{activity of native pancreatin} - \text{activity of HA-pancreatin}) / \text{activity of native pancrelipase}$$

Digestion difference is calculated by the following formula:

$$(\% \text{ of digestion of native pancreatin} - \% \text{ digestion of HA-pancreatin})$$

This *in vitro* test allows analysis of lipid, protein and carbohydrate digestion of HA-pancreatin. Lipase digestion is extremely sensitive to difference in enzymatic activities present in the reaction vessels hence to 10 % of difference in the activity corresponds to 10 % of difference in digestion amount. Digestion kinetics shows similar trends. These results suggest that native and HA-pancreatin have similar lipidic digestion pattern.

Protein digestion profiles are almost superimposable suggesting that protease activity of HA-pancreatin sustains the digestion at the same level of the native pancrelipase. A difference of about 60% of activity for protease does not produce any effects on protein digestion extent.

Carbohydrate digestion profiles are different since maltose production is lower in HA-pancreatin. The comparison of the difference in amylase activity and of the amount of digested products shows that in the HA-pancreatin also the amylolysis occurs.

Reference Example 10: Single step process

Preparation of HA-pancreatin; 0.1 g/mL; single step; scale up (SP= 38 -acetone : aqueous solvent = 35:65).

For this example a native pancrelipase (code 005A) with the following characteristics was used: lipase specific activity 81.1 IU /mg, protease activity 284.2 IU /mg and amylase activity 517.9 IU /mg; P/L ratio is 3.5 and A/L ratio is 6.4.

Step a1) Suspending- Precipitating: 100 g of native pancrelipase are dispersed in 1 L of a solvent mixture of buffer (pH=7, 10mM phosphate) and acetone (65 volumes of buffer and 35 volumes of acetone) under stirring for 60 minutes at 4°C.

Step a2) Separating: The mixture of step a1 is centrifuged (15 min at 2,700 g at 4°C) to separate supernatant from pellet, which contains the pancreatic enzymes.

Step a3) Drying: The pellets are dried with high efficiency pump at 0.2 mbar.

The material was analyzed. Table 22 reports lipase (L), specific activity measured for sample obtained using different scale.

Table 22

| Sample | Scale | LSA | LY | EF |
|---|---|---|---|---|
| 34 | pilot | 168.1 | 89 | 2.1 |
| 35 | Scale up | 176.2 | 81 | 2.2 |
| 36 | Scale up | 169.5 | 81 | 2.1 |
| LSA= lipase specific activity(IU /mg); EF= enrichment factor = LSA in pellet step a2 (IU /mg)/lipase activity in native raw material (IU /mg); LY = Lipase Yield (%). | | | | |

Table 22 shows the lipase activity of the final purified material obtained with the scaled-up process here applied. Results of this scale are coherent with pilot scale.

Table 23

| Sample | LSA | PSA | ASA | P/L ratio | A/L ratio |
|---|---|---|---|---|---|
| 35 | 176.2 | 260.3 | 169.2 | 1.48 | 0.96 |
| 36 | 169.5 | 265.0 | 179.6 | 1.56 | 1.06 |
| LSA= lipase specific activity (IU /mg); PSA = Protease specific activity (IU /mg); ASA = Amylase specific activity (IU /mg). | | | | | |

Good reproducibility in term of lipase, protease, amylase activity is obtained.

Example 1: Double precipitation process

Preparation of HA-pancreatin; 0.1 g/mL; double precipitation; pilot scale (SP(a1.1) = 38 -acetone: aqueous solvent = 35:65; SP(a1.3) = 36).

A native pancrelipase (code 005A) with the following characteristics was used: lipase specific activity 81.1 IU /mg, protease activity 284.2 IU /mg and amylase activity 517.9 IU /mg; P/L ratio is 3.5 and A/L ratio is 6.4.

Step a1.1- Suspending- precipitation: 10 g of native pancrelipase are dispersed in 100 mL of a solvent mixture of buffer (pH=7, 10mM phosphate) and acetone (65 volumes of buffer and 35 volumes of acetone) under stirring for 60 minutes at 4°C.

Step a1.2- Separating: The mixture of step a1 is centrifuged (10 min at 2,700 g at 4°C) to separate supernatant from pellet, which contains the pancreatic enzymes.

Step a3.1- Drying: The pellets are dried with high efficiency pump at 0.2 mbar.

Step a1.3- Precipitating: Acetone is added to the supernatant of step a1.2 (to reach SP of 36) under stirring for 60 minutes at 4°C.

Step a2- Separating: The mixture of step a4 is centrifuged (10 min at 2,700 g at 4°C) to separate supernatant from pellet, which contains the pancreatic enzymes.

Step a3.2- Drying: The pellets are dried with high efficiency pump at 0.2 mbar.

Step a4- Mixing: Dried pellets from step a3.1 and a3.2 are mixed together.

The material is analyzed. Table 24 reports lipase (L), protease (P) and amylase (A) specific activities measured for each sample.

Table 24.

| Sample | LSA | PSA | ASA | LY | PY | AY |
|---|---|---|---|---|---|---|
| 37 | 175.6 | 264.7 | 202.3 | 90 | 39 | 16 |
| 38 | 9.3 | 573.1 | 555.9 | 1 | 21 | 11 |
| 39 | 145.6 | 337.8 | 275.3 | 91 | 60 | 27 |
| LSA= lipase specific activity(IU /mg); PSA= protease specific activity(IU /mg); ASA= amylase specific activity (IU /mg). L= lipase; P= protease; A= amylase; A= activity; Y=yield (%); | | | | | | |

Example 2: Double precipitation process

Preparation of HA-pancreatin; 0.1 g/mL; double precipitation; scale up (SP(a1.1) = 38 - acetone: aqueous solvent = 35:65; SP(a1.3) = 36).
A native pancrelipase with the following characteristics was used: lipase activity 128.7 IU /mg, protease activity 324.6 IU /mg and amylase activity 408.0 IU /mg; P/L ratio is 2.5 and A/L ratio is 3.2.

Step a1.1- Suspending- precipitating: 100 g of native pancrelipase are dispersed in 1 L of a solvent mixture of buffer (pH=7, 10mM phosphate) and acetone (65 volumes of buffer and 35 volumes of acetone) under stirring for 60 minutes at 4°C.
Step a1.2- Separating: The mixture of step a1 is centrifuged (15 min at 2,700 g at 4°C) to separate supernatant from pellet, which contains the pancreatic enzymes.
Step a3.1- Drying: The pellets are dried with high efficiency pump at 0.2 mbar.
Step a1.3- Second precipitating: acetone is added to the supernatant of step a1.2 (SP of 36) for 120 or 180 minutes at 4°C in static condition.
Step a2- Separating: The mixture of step a1.3 is centrifuged (15 min at 2,700 g at 4°C) to separate supernatant from pellet, which contains the pancreatic enzymes.
Step a3.2- Drying: The pellets are dried with high efficiency pump at 0.2 mbar.
Step a4- Mixing: Dried pellets from step a3.1 and a3.2 are mixed together

The material is analyzed. Table 25 reports lipase (L), protease (P) and amylase (A) specific activities measured for each sample.

Table 25

| Sample | Step | Precipitation Time (min) | LSA | PSA | ASA | LY | PY | AY |
|---|---|---|---|---|---|---|---|---|
| 40 | a1.1 | 60 | 282.0 | 274.7 | 174.1 | 81 | 31 | 16 |

(continued)

| Sample | Step | Precipitation Time (min) | LSA | PSA | ASA | LY | PY | AY |
|--------|------|--------------------------|-------|-------|-------|----|----|----|
| 41 | A1.3 | 120 | 90.4 | 544.0 | 371.0 | 5 | 11 | 6 |
| 42 | A1.3 | 180 | 118.6 | 496.1 | 251.7 | 4 | 7 | 3 |
| LSA= lipase specific activity(IU /mg); PSA= protease specific activity(IU /mg); ASA= amylase specific activity (IU /mg). L= lipase; P= protease; A= amylase; A= activity; Y=yield (%); | | | | | | | | |

Reference Example 11: Single-step process

Preparation of HA-pancreatin; 0.1 g/mL; single-step; pilot scale (SP= 36- acetone: aqueous solvent = 43: 57)

For this example a native pancrelipase with the following characteristics has been used: lipase activity 85.5 IU /mg, protease activity 337.8 IU /mg and amylase activity 434.0 IU /mg; P/L ratio is 2.5 and A/L ratio is 3.2.

Step a1- Suspending- precipitating: 10 g of native pancrelipase are dispersed in 100 mL of a solvent mixture of buffer (pH=7, 10 mM phosphate) and acetone (57 volumes of buffer and 43 volumes of acetone) under stirring for 60 minutes at 4°C.

Step a2- Separating: The mixture of step a1 is centrifuged (10 min at 2,700 g at 4°C) to separate supernatant from pellet, which contains the pancreatic enzymes.

Step a3) -Drying: The pellets of step a2 are dried with high efficiency pump at 0.2 mbar.

The material is analyzed. The amount of pancrelipase, expressed as lipase activity is measured in pellet from step a3.

Table 26

| Sample | LSA | LY | EF |
|--------|-------|----|-----|
| 43 | 149.0 | 83 | 1.7 |
| 31 | 184.3 | 88 | 2.2 |
| LSA= lipase specific activity (IU /mg); L= lipase; Y=yield (%);EF= enrichment factor = LSA in pellet step a2 (IU /mg) /lipase activity in native raw material (IU /mg). | | | |

Results reported in Table 26 showed that SP=38 produced good results.

Reference Example 12: Single-step process

Preparation of HA-pancreatin; 0.1 g/mL; single-step; pilot scale (SP= 38- isopropyl-alcohol: aqueous solvent = 40: 60).

Step a1- Suspending- precipitation: 10 g of native pancrelipase was dispersed in 100 mL of a solvent mixture of buffer (pH=7, 10 mM phosphate) and isopropyl alcohol (60 volumes of buffer and 40 volumes ofisopropyl alcohol) under stirring for 60 minutes at 4°C.

Step a2- Separating: The mixture of step a1 is centrifuged (10 min at 2,700 g at 4°C) to separate supernatant from pellet, which contains the pancreatic enzymes.

Step a3- Drying: The pellets of step a2 are dried with high efficiency pump at 0.2 mbar.

Table 27

| Sample | LSA | EF | ASA |
|--------|-------|-----|-------|
| 44 | 162.0 | 1.8 | 200.7 |

(continued)

| Sample | LSA | EF | ASA |
|---|---|---|---|
| 27 | 222.7 | 2.4 | 158.9 |

| LSA= lipase specific activity(IU /mg); ASA= amylase specific activity(IU /mg); EF= enrichment factor = LSA in pellet step a2 (IU /mg)/lipase activity in native raw material (IU /mg). |
|---|

Results reported in Table 27 showed that acetone precipitation produced better results in terms of lipase activity and enhancement compared to isopropyl-alcohol.

Example 3: Ammonium Sulfate Precipitation

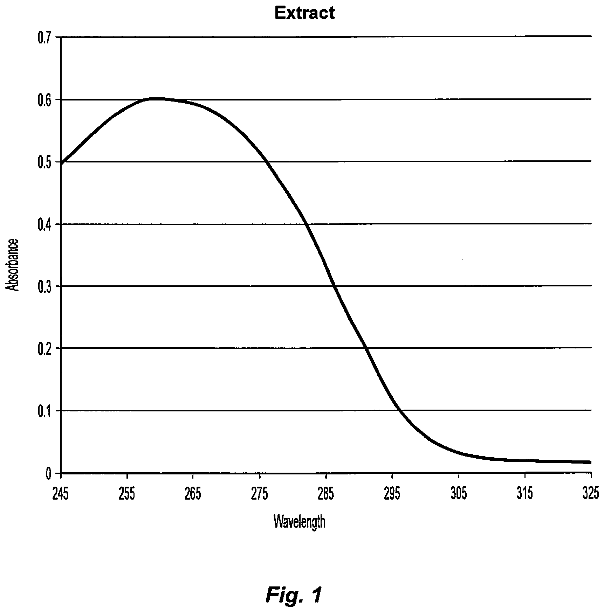

A water soluble extract of pancreatin was prepared through dispersion in cold phosphate buffered saline (PBS) to a concentration of 40 mg/mL. The dispersion was incubated on ice with occasional stirring and then centrifuged at 16,000xg for 5 minutes. The supernatant containing dissolved pancreatin was decanted. The supernatant absorbed strongly in the UV with an absorbance with a peak at 259 nm, indicative of the presence of dissolved nucleic acids. (Figure 1).

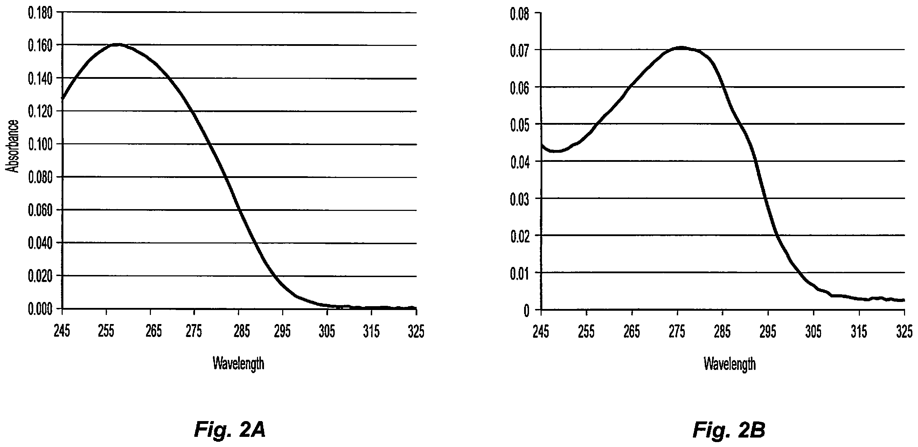

The supernatant was then mixed with saturated ammonium sulfate to a final concentration of about 60% saturated ammonium sulfate. The resulting precipitate was recovered by centrifugation and dissolved in phosphate buffered saline (PBS). The UV spectrum of a suitable dilution of the re-dissolved precipitate is shown in Figure 2B. The peak at 280 nm is typical of a protein solution. The supernatant had an absorbance peak at 260 nm, typical of dissolved nucleic acid. Comparison of the concentration and absorbance values (Figure 1 and Figure 2) indicates that about 60-90% of the material absorbing in the 260 nm region is removed. The position and shape of the spectra are consistent with enrichment of protein and/or peptide components. The supernatant containing the material not precipitated by ammonium sulfate (Figure 2A) has 260 nm/280 nm absorbance ratio of 1.7 compared to the 260 nm/280 nm absorbance ratio of 1.4 of the water-soluble pancreatin extract (Figure 1). This increase in the 260 nm/280 nm ratio is consistent with an enrichment of DNA and/or RNA in the material that is not precipitated by ammonium sulfate.

Without being bound by a particular theory, these results suggest that ammonium sulfate precipitation removes a substantial amount of DNA/RNA material from the protein component of pancreatin, i.e., the fraction which contains the amylase, lipase, and protease.

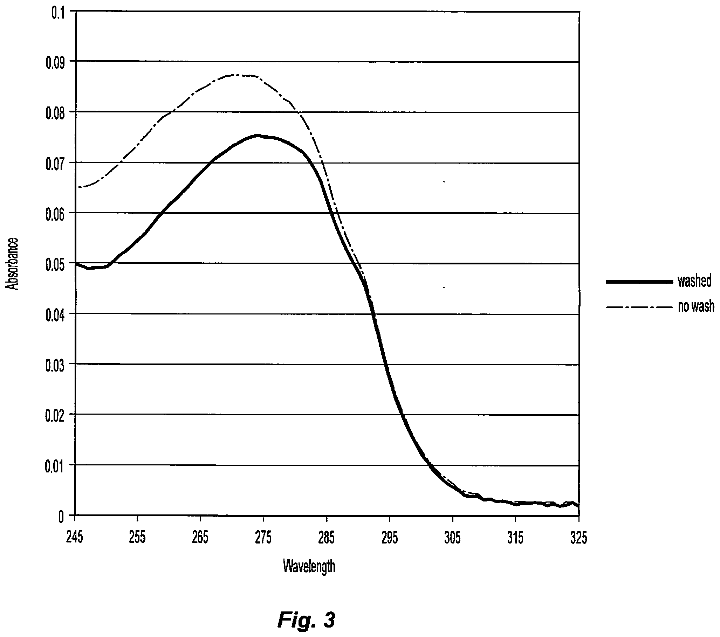

The pellet collected from the ammonium sulfate precipitation was washed with a 60% saturated ammonium sulfate solution. Spectra showed a peak shift from 272 nm to 275 nm following washing of the precipitate (Figure 3), indicating further removal of material with an absorbance peak at 260 nm.

The protein concentration of a solution can be estimated based on its absorbance at 280 nm. A protein assay (using bovine serum albumin as the standard) determined that a solution of 1.0 absorbance at 280 nm (path length 1 cm) corresponds to 0.19 mg/mL protein.

The pancreatin original extract contained 40 mg/mL, of which 0.5 mL was mixed with 0.75 mL saturated ammonium sulfate to form the precipitate as described in the experimental section. This precipitate was then resolublized in 1 mL PBS. The absorbance of 5 $\mu$L of the solution of re-solubilized precipitate diluted in 1.2 mL PBS was measured. Figure 3 shows the spectra of unwashed and washed ammonium sulfate precipitates, the recorded absorbance values at 280 nm are approximately 0.085 and 0.075 (1 cm path length) respectively after dilution (1/240).

As 1.0 absorbance corresponds to 0.19 mg/mL protein, the unwashed pellet has a protein concentration of 3.9 mg/mL (240 x 0.085 x 0.19) and the washed pellet has a protein concentration of 3.4 mg/mL. Since 20 mg of material (0.5 mL of a solution containing 40 mg/mL) was taken through the purification process, this represents a purification of approximately 20/3.9 = 5 fold. The activities of lipase and amylase in the protein-enriched precipitate were determined. As seen in Table 28 and 29, an average of 81% of the lipase activity and 83% of the amylase activity is recovered in the unwashed pellet. Washing the pellet with 60% ammonium sulfate reduces lipase recovery to 68%. These percentages are normalized to activity levels in the original water-soluble fraction of pancrelipase.

Table 28

| | Unwashed pellet | | | | Washed Pellet | | |
|---|---|---|---|---|---|---|---|
| | Recovery, % | | UV peak, nm | | Recovery, % | | UV peak, nm |
| | Lipase | UV | | | Lipase | UV | | notes |
| | 104 | 37 | 272.0 | | | | | |

(continued)

| | Unwashed pellet | | | | Washed Pellet | | | |
| | Recovery, % | | UV peak, nm | | Recovery, % | | UV peak, nm | |
| | Lipase | UV | | | Lipase | UV | | notes |
|---|---|---|---|---|---|---|---|---|
| | 60 | | | | | | | |
| | 103 | | | | | | | |
| | 72 | | | | | | | |
| | 163 | | | | | | | 1 |
| | 75 | 32 | 274.0 | | 68 | 24 | 276.7 | |
| | | | | | | 27 | 275.3 | 2 |
| | | | | | 73 | 25 | 275.6 | |
| | | | | | 69 | | | |
| | 65 | 33 | 270.0 | | 60 | 30 | 274.0 | |
| | 70 | 28 | 273.0 | | | | | |
| | 80 | | | | | | | 3 |
| | 83 | | | | | | | 3 |
| | 72 | | | | | | | 3 |
| | 75 | | | | | | | |
| | | | | | 88 | | | |
| | 108 | | | | | | | |
| | 73 | | | | | | | 4 |
| | 87 | | | | | | | 4 |
| | | | | | | | | |
| ave | 81 | 33 | 272.3 | | 72 | 27 | 275.4 | |
| stdev | 17 | 3.7 | 1.7 | | 10.3 | 2.6 | 1.1 | |

Lipase assays are recovery compared to extract
UV material is the integrated area from 245-325 nm and is the recovery compared to extract
Peak values are the UV peak for the ammonium sulfate fraction
Lipase was assayed within 5 hours of extract
1. Lipase >> 100%, data excluded
2. Plate reader malfunction, no enzyme assays that day.
3. Data not included in average. Pellet resuspended in a more concentrated volume than typical, recovery volume was significantly larger than usual.
4. Volume correction applied, pellet resuspended in a more concentrated volume than typical.

Table 29

| Amylase Activity of Ammonium Sulfate Fraction | | | |
|---|---|---|---|
| % Recovery Compared to Extract | | | |
| | | | |
| | | unwashed pellet | washed pellet |
| | | 124 | |
| | | 53 | |

(continued)

|  |  | unwashed pellet | washed pellet |
|---|---|---|---|
|  |  | 83 |  |
|  |  | 84 | 82 |
|  |  | 78 |  |
|  |  | 75 |  |
|  |  |  |  |
|  | average | 83 |  |
|  | Standard Deviation | 23 |  |

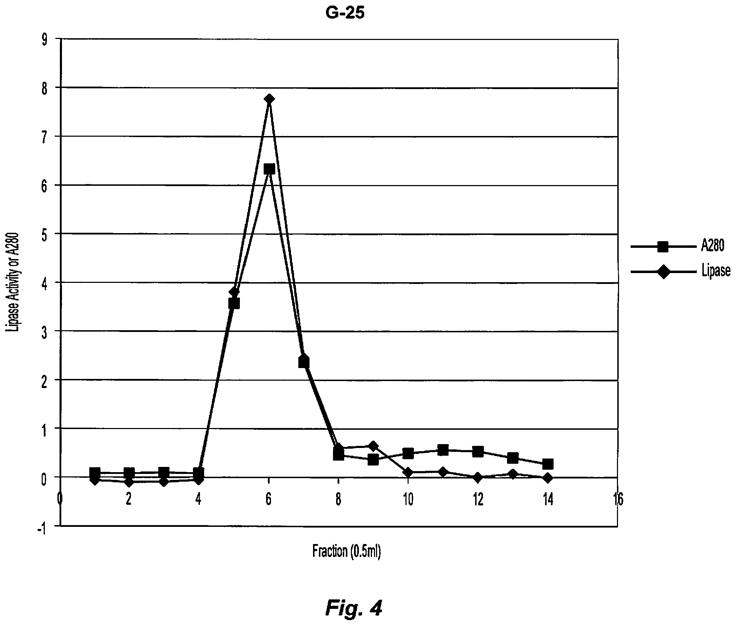

Prior to determining the activity of the pellet on a per milligram basis, the precipitate was desalted with a suitable column. Gel filtration chromatography was performed to desalt the ammonium sulfate precipitation product. The unwashed ammonium sulfate pellet was resuspended in two volumes of PBS buffer and 0.5 mL of the resulting suspension was applied to a G-25 Sephadex column and eluted by gravity. The elution profile of the ammonium sulfate fraction is presented in Figure 4. Fractions 4-8 show absorption at 280 nm, indicating the presence of protein. The methylumbelliferone assay to measure the activity of lipase also showed activity at fractions 4-8.

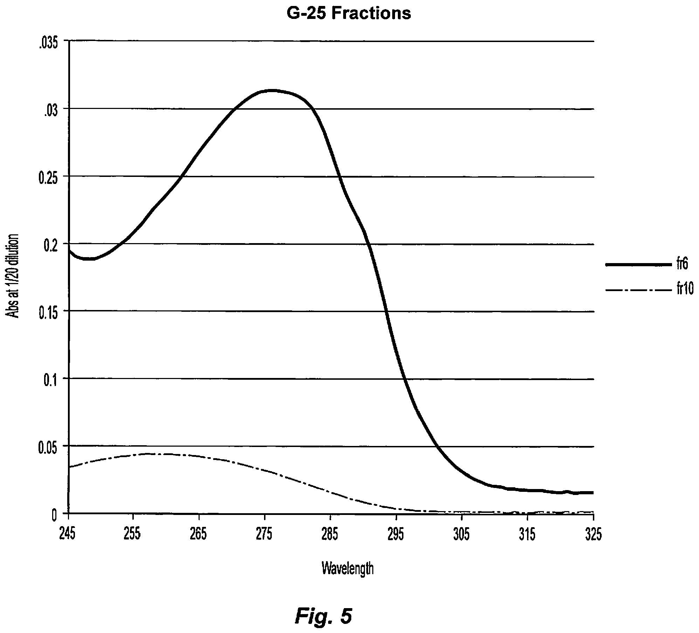

The later eluting material (fraction 10, Figure 5, scanned at a 1/20 dilution in water in a 1 cm cuvette) has an absorbance maximum of 260 nm, consistent with the absorbance profile of DNA. This suggests that, in addition to desalting, the G-25 column could be used to remove residual amounts of the 260 nm UV material (e.g., DNA).

Example 4: Ammonium Sulfate Precipitation

Desalting can also be performed using G-25 sephadex spin columns. Dry weight recovery can be done without concentrating the desalted material.

A 60% saturated ammonium sulfate fraction was prepared as described above in Examples, the ammonium sulfate pellet was resuspended in one volume of PBS buffer (compared to extract). A portion of the ammonium sulfate fraction was desalted over two G-25 sephadex spin columns equilibrated in water as described previously. One mL of the desalted material (equivalent to 37 mg of pancreatin) was precipitated with 2 volumes of isopropanol to aid subsequent drying and centrifuged for 5 minutes at 16K. The supernatant was removed and the pellet air dried at room temperature overnight, and 7.9 mg of solid was recovered.

A loss of approximately 17% of the UV-absorbing material was observed during the isopropanol precipitation. If the UV-absorbing material represents protein mass equivalent to that in the pellet, then correcting for this would mean that the desalted material contained 9.5 mg of solid. This represents a 37/9.5 = 3.9 fold concentration of the pancreatin starting material. Assuming lipase and amylase activities of 78% and 83% (Table 28 and 29), with respect to the extract, then this represents an increase in specific activity of about 3 with respect to lipase and amylase. It has been observed that 90% of the lipase activity is recovered during the extraction procedure, thus the increase in specific activity would be 2.7 fold. The drying of the recovered material was not complete, however, and a further increase in specific activity is anticipated on further drying.

A repeat of the dry weight determination was performed with additional drying with higher heat. A 60% saturated ammonium sulfate fraction was prepared as described in the above, the ammonium sulfate pellet was resuspended in one volume of PBS buffer (compared to extract). A portion of the ammonium sulfate fraction was desalted over two G-25 sephadex spin columns equilibrated in water as described in the experimental section. One mL of the desalted material (equivalent to 37.5 mg of pancreatin) was precipitated with 2 volumes of isopropanol to aid subsequent drying and centrifuged for 5 minutes at 16K. The supernatant was removed and the pellet air dried at 80°C overnight; 4.6 mg of solid was recovered.

A loss of approximately 17% of the UV-absorbing material was observed during the isopropanol precipitation. If this UV-absorbing material represents protein mass equivalent to that in the pellet then correcting for this would mean the desalted material contained 5.4 mg of solid. This represents a 37.5/5.4 = 7-fold concentration of the pancreatin starting material. Lipase and amylase activities were 74% and 78%, with respect to the extract. This represents an increase in specific activity of about 5 with respect to lipase and amylase. It has been observed that 90% of the lipase activity is recovered during the extraction procedure, thus the increase in specific activity would be 4.5 fold.

Lipase recoveries were 87-103% in columns equilibrated with PBS, while lipase recoveries were 52-72% in columns equilibrated with water, suggesting that greater enzyme activity is preserved when the columns were equilibrated with PBS as compared to water.

Longer column run times also showed evidence of autohydrolysis of proteins, presumably by the proteases present. This may be attenuated through the addition of protease inhibitors or through adjusting conditions such as pH and temperature.

It is preferable to add components to stabilize the proteins during processing, including the final drying step. These stabilizers could include salts, carbohydrates, antioxidants, polymers and protease inhibitors such as EDTA and soybean trypsin inhibitor. A salt solution of 5 mM phosphate buffered at pH 7.4 would function to maintain the pH and possibly bind calcium, which would inhibit proteolysis. The final drying step following isopropanol precipitation was found to result in significant reductions in lipase activity. It may be preferable to freeze dry the final material.

Example 5: Ammonium Sulfate Precipitation

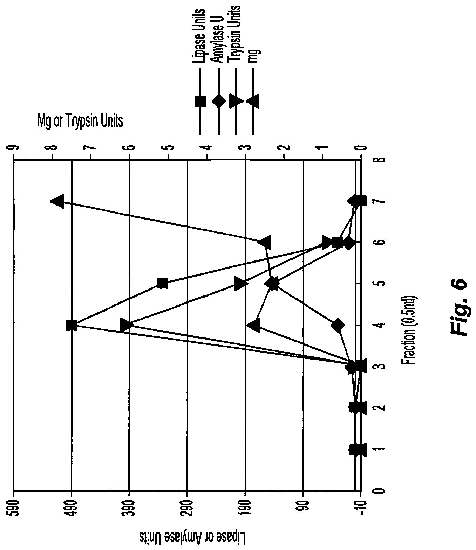

Ammonium sulfate extraction and precipitation were performed as described above in a bench top centrifuge at 4°C. The resulting solid was desalted on s G-25 sephadex spin column as described previously in Examples. The 4.5 mL G-25 column was loaded with 0.5 mL of resuspended precipitate. Assuming all of the pancreatin was precipitated by ammonium sulfate, the resuspended precipitate would be estimated to contain 16 mg of pancreatin. Figure 6 shows the activity of lipase, amylase, and trypsin in each fraction, as well as the weight, in mg, of material recovered. After extraction, precipitation with ammonium sulfate and desalting over the G-25 column approximately 5 mg of solid was recovered in fractions 4 and 5. This yields a calculation of a lipase specific activity increase of about 3 fold, amylase activity increase of about 1.7 fold, and a trypsin activity increase of about 2 fold in fractions 4 and 5 compared to the original pancreatin aqueous suspension/solution.

When desalting was performed on two desalting columns, with 13.4 mg of material from 33.3 mg of starting material, the specific activity of lipase increased by about 1.2 fold and the specific activity of amylase increased by about 1.5 fold. Without being bound by a particular theory, this decrease in activity may be due to removal of important stabilizing salts by the extended desalting process of the extended processing time.

The foregoing detailed description has been given for clearness of understanding only and no unnecessary limitations should be understood there from as modifications will be obvious to those skilled in the art. While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth and as follows in the scope of the appended claims.

**Claims**

1. A process for the preparation of mammalian-derived HA-pancreatin including lipases, proteases and amylases, having specific lipase activity of at least 120 IU/mg, comprising:

   a1.1) suspending starting mammalian pancreatin and precipitating a first insoluble portion in a solvent having a Hildebrand solubility parameter of $38(MPa)^{0.5}$;
   a1.2) separating the soluble portion of step a1.1 from the first insoluble portion;
   a1.3) precipitating a second insoluble portion by adding to the soluble portion of step a1.2 at least one organic solvent thereof to obtain a mixture having a Hildebrand solubility parameter of 36;
   a2) separating the second insoluble portion of step a 1.3 from the soluble portion;
   a3.1) drying the first insoluble portion of step a1 .2;
   a3.2) drying the second insoluble portion of step a2;
   a4) mixing together the first insoluble portion of step a3.1 with the second insoluble portion of step a3.2, wherein

      (i) said solvent used in step a1.1 is a mixture of acetone and buffer solution having a pH of 7,
      (ii) the process temperature is 4°C,
      (iii) the mixture of step a1.3 is a mixture of acetone and buffer solution having a pH of 7, and
      (iv) the pancreatin of step a1.1 is in an amount of 0.1 g/ml.

2. The process of claim 1 for the preparation of pancreatin having specific lipase activity of at least 150 IU/mg,

3. The process of claim 1 or claim 2 for the preparation of pancreatin having specific lipase activity of at least 200 IU/mg.

4. The process of any one of claims 1 to 3, for the preparation of pancreatin having specific lipase activity of at least 400 IU/mg.

5. The process of claim 1, comprising the step of microbial and /or viral load reduction.

6. The process of claim 5, wherein the bacterial and/or viral load reduction is carried out by filtration, heating, ionizing radiation, high pressure or by alkylation.

**Patentansprüche**

1. Verfahren zur Herstellung von von Säugetieren stammendem HA-Pankreatin, das Lipasen, Proteasen und Amylasen einschließt, mit einer spezifischen Lipaseaktivität von mindestens 120 IE/mg, umfassend:

a1.1) das Suspendieren von Ausgangssäugetierpankreatin und das Ausfällen eines ersten unlöslichen Teils in einem Lösungsmittel mit einem Hildebrand-Löslichkeitsparameter von 38 $(MPa)^{0,5}$,
a1.2) das Abtrennen des löslichen Teils von Schritt a1.1 von dem ersten unlöslichen Teil,
a1.3) das Ausfällen eines zweiten unlöslichen Teils durch Zugabe des löslichen Teils von Schritt a1.2 zu mindestens einem organischen Lösungsmittel davon unter Erhalt einer Mischung mit einem Hildebrand-Löslichkeitsparameter von 36,
a2) das Abtrennen des zweiten unlöslichen Teils von Schritt a1.3 von dem löslichen Teil,
a3.1) das Trocknen des ersten unlöslichen Teils von Schritt a1.2,
a3.2) das Trocknen des zweiten unlöslichen Teils von Schritt a2,
a4) das Mischen des ersten unlöslichen Teils von Schritt a3.1 mit dem zweiten unlöslichen Teil von Schritt a3.2, wobei

(i) es sich bei dem in Schritt a1.1 verwendeten Lösungsmittel um eine Mischung von Aceton und einer Pufferlösung mit einem pH-Wert von 7 handelt,
(ii) die Verfahrenstemperatur 4 °C beträgt,
(iii) es sich bei der Mischung von Schritt a1.3 um eine Mischung von Aceton und einer Pufferlösung mit einem pH-Wert von 7 handelt und
(iv) das Pankreatin von Schritt a1.1 in einer Menge von 0,1 g/ml vorliegt.

2. Verfahren nach Anspruch 1 zur Herstellung von Pankreatin mit einer spezifischen Lipaseaktivität von mindestens 150 IE/mg.

3. Verfahren nach Anspruch 1 oder Anspruch 2 zur Herstellung von Pankreatin mit einer spezifischen Lipaseaktivität von mindestens 200 IE/mg.

4. Verfahren nach einem der Ansprüche 1 bis 3 zur Herstellung von Pankreatin mit einer spezifischen Lipaseaktivität von mindestens 400 IE/mg.

5. Verfahren nach Anspruch 1, umfassend den Schritt der Reduktion der mikrobiellen und/oder viralen Belastung.

6. Verfahren nach Anspruch 5, wobei die Reduktion der bakteriellen und/oder viralen Belastung durch Filtration, Erhitzen, ionisierende Strahlung, hohen Druck oder durch Alkylierung erfolgt.

**Revendications**

1. Procédé de préparation de HA-pancréatine dérivée de mammifère incluant des lipases, des protéases et des amylases, ayant une activité lipase spécifique d'au moins 120 UI/mg, comprenant :

a1.1) la suspension de la pancréatine mammalienne de départ et la précipitation d'une première portion insoluble dans un solvant ayant un paramètre de solubilité de Hildebrand de 38 $(MPa)^{0,5}$ ;
a1.2) la séparation de la portion soluble de l'étape a1.1 d'avec la première portion insoluble ;
a1.3) la précipitation d'une seconde portion insoluble par ajout à la portion soluble de l'étape a1.2 d'au moins un solvant organique de celle-ci pour obtenir un mélange ayant un paramètre de solubilité de Hildebrand de 36 ;

a2) la séparation de la seconde portion insoluble de l'étape a1.3 d'avec la portion soluble ;

a3.1) le séchage de la première portion insoluble de l'étape a1.2 ;

a3.2) le séchage de la seconde portion insoluble de l'étape a2 ;

a4) le mélangeage de la première portion insoluble de l'étape a3.1 avec la seconde portion insoluble de l'étape a3.2, dans lequel

(i) ledit solvant utilisé dans l'étape a1.1 est un mélange d'acétone et de solution tampon ayant un pH de 7,

(ii) la température du procédé est de 4 °C,

(iii) le mélange de l'étape a1.3 est un mélange d'acétone et de solution tampon ayant un pH de 7, et

(iv) la pancréatine de l'étape a1.1 est dans une quantité de 0,1 g/ml.

2. Procédé selon la revendication 1 pour la préparation de pancréatine ayant une activité lipase spécifique d'au moins 150 UI/mg.

3. Procédé selon la revendication 1 ou la revendication 2 pour la préparation de pancréatine ayant une activité lipase spécifique d'au moins 200 UI/mg.

4. Procédé selon l'une quelconque des revendications 1 à 3, pour la préparation de pancréatine ayant une activité lipase spécifique d'au moins 400 UI/mg.

5. Procédé selon la revendication 1, comprenant l'étape de réduction de la charge microbienne et/ou virale.

6. Procédé selon la revendication 5, dans lequel la réduction de la charge bactérienne et/ou virale est mise en œuvre par filtration, chauffage, rayonnements ionisants, pression élevée ou alkylation.

**Extract**

*Fig. 1*

*Fig. 2B*

*Fig. 2A*

Fig. 3

**G-25**

Fig. 4

EP 3 003 360 B1

**G-25 Fractions**

*Fig. 5*

EP 3 003 360 B1

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6051220 A
- US 20040057944 A
- US 20010046493 A
- WO 2006044529 A

**Non-patent literature cited in the description**

- **HWANG et al.** *Ind. Eng. Chem. Res.,* 2007, vol. 46, 4289
- **BARTON.** Handbook of Solubility Parameters. CRC Press, 1983